(19) Europäisches Patentamt
European Patent Office
Office européen des brevets

(11) EP 3 937 949 B1

(12) **FASCICULE DE BREVET EUROPEEN**

(45) Date de publication et mention de la délivrance du brevet:
**27.09.2023 Bulletin 2023/39**

(21) Numéro de dépôt: **20710511.5**

(22) Date de dépôt: **12.03.2020**

(51) Classification Internationale des Brevets (IPC):
***A61K 31/56*** *(2006.01)* ***A61K 31/57*** *(2006.01)*
***A61K 31/575*** *(2006.01)* ***A61K 31/58*** *(2006.01)*
***A61P 11/00*** *(2006.01)*

(52) Classification Coopérative des Brevets (CPC):
**A61K 31/56; A61K 31/57; A61K 31/575; A61K 31/58; A61P 11/00**

(86) Numéro de dépôt international:
**PCT/EP2020/056591**

(87) Numéro de publication internationale:
**WO 2020/187679 (24.09.2020 Gazette 2020/39)**

(54) **PHYTOECDYSONES ET LEURS DÉRIVÉS POUR LEUR UTILISATION DANS LE TRAITEMENT DE L'ALTÉRATION DE LA FONCTION RESPIRATOIRE**

PHYTOECDYSONE UND DEREN DERIVATE ZUR VERWENDUNG BEI DER BEHANDLUNG VON BEEINTRÄCHTIGTER LUNGENFUNKTION

PHYTOECDYSONES AND THE DERIVATIVES THEREOF FOR USE IN THE TREATMENT OF IMPAIRED LUNG FUNCTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.03.2019 FR 1902727**

(43) Date de publication de la demande:
**19.01.2022 Bulletin 2022/03**

(73) Titulaires:
- **Biophytis**
  **75001 Paris (FR)**
- **SORBONNE UNIVERSITE**
  **75006 Paris (FR)**

(72) Inventeurs:
- **LATIL, Mathilde**
  **75019 PARIS (FR)**
- **DILDA, Pierre**
  **75016 PARIS (FR)**
- **LAFONT, René**
  **75016 PARIS (FR)**
- **VEILLET, Stanislas**
  **91600 SAVIGNY SUR ORGE (FR)**

(74) Mandataire: **Ipside**
**6, Impasse Michel Labrousse**
**31100 Toulouse (FR)**

(56) Documents cités:
**WO-A1-2015/177469 WO-A1-2018/197708**
**RU-C2- 2 257 906**

- **DATABASE EMBASE [Online] ELSEVIER SCIENCE PUBLISHERS, AMSTERDAM, NL; 1998, WU X ET AL: "The effect of ecdysterone on rat lung reperfusion injury", XP002796366, Database accession no. EMB-1998281055 & WU X ET AL: "The effect of ecdysterone on rat lung reperfusion injury", CHINESE PHARMACOLOGICAL BULLETIN 1998 PUBLICATION CENTRE OF ANHUI MEDICAL UNIVERSITY CHN, vol. 14, no. 3, 1998, pages 256-258, ISSN: 1001-1978**
- **WU XU ET AL.: "Effect of Ecdysterone on Lung Contusion from Impact", CHINESE JOURNAL OF TRAUMATOLOGY, mai 1997 (1997-05), pages 295-296, XP002796367,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **DILDA P ET AL: "P.311BIO101 demonstrates combined beneficial effects on skeletal muscle and respiratory functions in a mouse model of Duchenne muscular dystrophy", NEUROMUSCULAR DISORDERS, ELSEVIER LTD, GB, vol. 29, 29 septembre 2019 (2019-09-29), XP085845011, ISSN: 0960-8966, DOI: 10.1016/J.NMD.2019.06.425 [extrait le 2019-09-29]**

## Description

### Domaine technique de l'invention

L'invention relève de l'utilisation de phytoecdysones et de dérivés hémi-synthétiques de phytoecdysones pour le traitement de l'altération de la fonction respiratoire, particulièrement dans le cadre de pathologies neuromusculaires.

### Technique antérieure

Les maladies neuromusculaires sont caractérisées par une altération du fonctionnement des unités motrices, composées de motoneurones, jonctions neuromusculaires et muscles squelettiques. Au-delà de l'altération de la fonction motrice des patients atteints par ces pathologies, de très nombreuses maladies neuromusculaires aigues ou évolutives conduisent à un dysfonctionnement des muscles respiratoires qui, à son tour, peut conduire à insuffisance respiratoire, une pneumonie et au décès des patients. En effet, les troubles respiratoires représentent la principale cause de mortalité chez les patients atteints de maladies neurologiques (Miller et al., 2009).

Les patients atteints de maladies neuromusculaires sont susceptibles de développer une altération de la fonction respiratoire, qui peut s'exprimer de manière évidente, avec la survenue fréquente d'infections comme des pneumonies ou des bronchites (principalement dues à l'inefficacité de la toux), l'impression de souffle court, des difficultés à expectorer mais, dans certains cas, les manifestations sont moins évidentes et les patients ont une perte d'appétit avec un amaigrissement important, des maux de tête, des sueurs ou une grande fatigue. Par conséquent, il est primordial de détecter le plus tôt possible l'atteinte respiratoire.

La prise en charge des problèmes respiratoires des patients atteints de pathologies neuromusculaires s'est considérablement améliorée ces dernières années, permettant ainsi d'augmenter la durée de vie des patients, comme par exemple les enfants atteints de maladie de Duchenne. Une surveillance régulière des paramètres respiratoires est mise en place à l'aide d'examens cliniques, de l'imagerie et de la spirométrie permettant l'exploration fonctionnelle respiratoire ou par gazométrie afin d'évaluer la qualité des échanges gazeux (mesure des taux d'oxygène et de gaz carbonique dans le sang artériel). Ces bilans réguliers permettent ainsi de déceler les conséquences de la faiblesse musculaire et de la dysfonction pulmonaire et, ainsi, permettent d'adapter la prise en charge médicale de ces patients pour compenser leur fonction pulmonaire défaillante et améliorer leur qualité de vie (Birnkrant et al., 2007 ; Finder et al., 2004 ; McKim et al., 2011). La prise en charge peut s'effectuer à plusieurs niveaux : elle peut viser à entretenir la mobilité et la souplesse de l'appareil respiratoire (kinésithérapie respiratoire par des mobilisation actives ou passives ou par hyper insufflations mécaniques) ou à désencombrer les voies respiratoires afin d'évacuer les sécrétions produites par les bronches (toux assistée ou drainage bronchique) ou, enfin, lorsque la ventilation naturelle ne répond plus aux besoins de l'organisme, de suppléer la respiration des patients par une ventilation non invasive ou, dans les cas les plus extrêmes, par une ventilation par trachéotomie.

Les atteintes du cortex, du tronc cérébral, de la moelle épinière, des motoneurones, des nerfs périphériques, des jonctions neuromusculaires ou des muscles peuvent toutes conduire à un dysfonctionnement du système respiratoire.

Il existe de nombreuses causes de maladies musculaires chroniques entraînant un dysfonctionnement des muscles respiratoires, y compris les myopathies (congénitales, héréditaires ou acquises), les myasthénies ou les myotonies.

Pour exemple, dans la dystrophie musculaire de Duchenne (DMD), la défaillance respiratoire, entraînant de nombreuses complications pulmonaires, est la cause de la majorité des décès observés chez les patients DMD (Mayer et al., 2015 ; Vianello et al., 1994 ; Baydur et al., 1990 ; Smith et al., 1987 ; Inkley et al., 1974). La DMD est la plus fréquente des dystrophies musculaires. Elle touche un garçon sur 3500 et résulte de mutations affectant le gène de la dystrophine, localisé sur le chromosome X. Une forme moins sévère, la dystrophie musculaire de Becker (BMD), implique également le gène de la dystrophine et touche un garçon sur 18000. Les garçons atteints de DMD n'ont généralement pas de difficultés à respirer ou à tousser lorsqu'ils sont encore en mesure de marcher. À mesure qu'ils vieillissent et que la maladie affecte leurs muscles respiratoires, les garçons atteints risquent de contracter des infections respiratoires, souvent à cause d'une toux inefficace. Les cellules musculaires lisses et, par extension, les muscles lisses des voies aériennes sont impliqués dans de nombreuses pathologies respiratoires.

Outre le processus myopathique, les anomalies du système pulmonaire lui-même (voies respiratoire ou poumons) sont fortement impliquées dans la défaillance ventilatoire chez les patients DMD (Benditt et Boitano, 2013). En effet, la compliance pulmonaire (capacité du poumon à modifier son volume en réponse à une variation de pression) est diminuée pour deux raisons principales : la rétractation et l'affaissement des alvéoles pulmonaires (atélectasie) causée par l'hypoventilation, mais également l'apparition de fibrose et l'obstruction des voies respiratoires, ayant pour conséquence d'augmenter la résistance des voies respiratoires (Lo Mauro et Aliverti, 2016).

Les propriétés élastiques du poumon sont modifiées chez les patients atteints de dystrophie musculaire, le poumon devenant moins distensible. La cause de la réduction de la distensibilité pulmonaire dans la dystrophie musculaire n'est pas encore totalement connue. Néanmoins, différentes hypothèses ont été proposées pour tenter d'expliquer la diminution de l'élasticité des poumons : une maturation incomplète du tissu pulmonaire dans le cadre d'une maladie congénitale, l'atélectasie induite par l'hypoventilation, l'augmentation de la tension superficielle alvéolaire, ou une altération du parenchyme pulmonaire due à une fibrose. De plus, un des facteurs importants pouvant expliquer cette réduction de la distensibilité et de la compliance pulmonaire est une respiration à faible volume pulmonaire, ce qui est une caractéristique des dystrophies musculaires (Lo Mauro et Aliverti, 2016). Un autre mécanisme impliqué est la dégradation chronique et progressive des muscles respiratoires qui limitent, *de facto,* la gamme d'activité du poumon. En effet, les propriétés élastiques d'un système sont en partie déterminées par les contraintes auxquelles le système est soumis. La capacité pulmonaire totale est le résultat de la balance entre la pression de rétraction élastique instantanée pendant la ventilation et la pression générée par la contraction des muscles inspiratoires. Cette dernière est réduite et, par conséquent, la capacité pulmonaire totale également, ce qui altère les paramètres d'expiration et provoque la diminution de la compliance pulmonaire.

Pour conclure, l'insuffisance respiratoire, caractérisée par l'incapacité du système respiratoire à fournir une oxygénation et une élimination du dioxyde de carbone adéquates, est commune chez les patients atteints de DMD.

Par conséquent, l'évaluation du dysfonctionnement du système respiratoire chez la souris mdx, le modèle murin de la myopathie de Duchenne le plus utilisé, est un paramètre important à considérer dans la mise en place et l'évaluation de solutions thérapeutiques dans le cadre de maladies neuromusculaires.

L'évaluation du système respiratoire de souris mdx au cours des études précliniques présente des avantages significatifs : d'une part, il s'agit d'un déficit cliniquement important et d'autre part, les mesures spirométriques ou pléthysmographiques sont non invasives et peuvent être répétées de manière longitudinale au cours de l'essai et éventuellement effectuées en tant que critère d'évaluation de l'efficacité de divers traitements de la dystrophie musculaire. Différents groupes se sont intéressés à la fonction respiratoire chez la souris mdx en comparaison avec des souris contrôles saines, et ont rapporté les altérations de la fonction respiratoire chez la souris mdx (Gosselin et al. 2003 ; Matecki et al. 2003 ; Mosqueira et al. 2013 ; Gayraud et al., 2007). Ainsi, avec quelques modifications sur la sévérité des altérations et l'âge d'apparition de ces altérations, tous rapportent une altération des paramètres respiratoires, en conditions normoxiques (Huang et al., 2011) ou bien en réponse à l'hypercapnie (Gosselin et al. 2003).

Le document RU 2 257 906 décrit une composition comprenant de l'ecdysterone (20hydroxyecdysone) pour son utilisation dans le traitement de maladies pulmonaires et pleurales suppurées.

WU X ET AL: CHINESE PHARMACOLOGICAL BULLETIN, vol. 14, no. 3, 1998, pages 256-258 décrit l'effet de l'ecdysterone sur les lésions de reperfusions pulmonaire chez le rat.

WU XU ET AL.:CHINESE JOURNAL OF TRAUMATOLOGY, mai 1997 (1997-05), pages 295-296 décrit l'effet de l'ecdysterone sur la contusion pulmonaire due à un impact.

## Présentation de l'invention

Les inventeurs ont découvert que les phytoecdysones et des dérivés hémi-synthétiques de phytoecdysones améliorent de manière significative la fonction respiratoire de mammifères atteints par une maladie neuromusculaire, en limitant au cours du temps l'altération des paramètres respiratoires, ainsi qu'en améliorant les paramètres mécaniques de l'appareil respiratoire. Les paramètres respiratoires et les paramètres mécaniques de l'appareil respiratoire sont déterminés respectivement par pléthysmographie sur corps entier sur des animaux vigiles et par ventilateur à piston commandé par unité centrale de commande (communément appelé « ordinateur ») sur des animaux anesthésiés, ledit ventilateur utilisant la technique de l'oscillation forcée comme l'appareil connu sous le nom FlexiVent™. Ces effets montrent une amélioration de la fonction respiratoire chez les mammifères atteints de pathologies neuromusculaires génétiques ou acquises. Les phytoecdysones représentent une importante famille de phytostérols polyhydroxylés structuralement apparentés aux hormones de mue des insectes. Ces molécules sont produites par de nombreuses espèces végétales et participent à leur défense contre les insectes ravageurs. La phytoecdysone majoritaire est la 20-hydroxyecdysone.

À cet effet, l'invention concerne au moins une phytoecdysone et/ou au moins un dérivé hémi-synthétique de phytoecdysone, pour leur utilisation dans le traitement d'une altération de la fonction respiratoire, comme définies dans la revendication 1.

De préférence l'invention concerne une composition comprenant au moins une phytoecdysone et/ou au moins un dérivé hémi-synthétique de phytoecdysone, pour son utilisation dans le traitement d'une altération de la fonction respiratoire, comme définies dans la revendication 1.

Dans des modes particuliers de réalisation, l'invention répond en outre aux caractéristiques suivantes, mises en oeuvre séparément ou en chacune de leurs combinaisons techniquement opérantes.

Les phytoecdysones et leurs dérivés sont avantageusement purifiés au grade pharmaceutique.

Une phytoecdysone utilisable selon l'invention est par exemple la 20-hydroxyecdysone et un dérivé hémi-synthétique de phytoecdysone utilisable est par exemple un dérivé hémi-synthétique de 20-hydroxyecdysone.

A cet effet, selon un mode particulier de réalisation, la composition comporte de la 20-hydroxyecdysone et/ou au moins un dérivé hémi-synthétique de 20-hydroxyecdysone.

La 20-hydroxyecdysone et ses dérivés sont avantageusement purifiés au grade pharmaceutique.

La 20-hydroxyecdysone utilisée est de préférence sous forme d'un extrait de végétaux riches en 20-hydroxyecdysone ou d'une composition comportant à titre d'agent actif la 20-hydroxyecdysone. Des extraits de végétaux riches en 20-hydroxyecdysone sont par exemple des extraits de *Stemmacantha carthamoides* (aussi appelée *Leuzea carthamoides), Cyanotis arachnoidea* et *Cyanotis vaga.*

Les extraits obtenus sont de préférence purifiés au grade pharmaceutique.

Dans un mode de réalisation la 20-hydroxyecdysone est sous forme d'extrait de plante ou d'une partie de plante, ladite plante étant choisie parmi les végétaux contenant au moins 0,5% de 20-hydroxyecdysone en poids sec dudit végétal, ledit extrait comportant au moins 95%, et de préférence au moins 97%, de 20-hydroxyecdysone. Ledit extrait est de préférence purifié au grade pharmaceutique. Ledit extrait est appelé par la suite BIO101. Il comporte de façon remarquable entre 0 et 0,05 %, en poids sec de l'extrait, d'impuretés, comme des composés mineurs, susceptibles d'affecter l'innocuité, la disponibilité ou l'efficacité d'une application pharmaceutique dudit extrait.

Selon un mode de réalisation de l'invention, les impuretés sont des composés à 19 ou 21 atomes de carbone, tels que la Rubrostérone, la Dihydrorubrostérone ou la Poststérone.

La plante à partir de laquelle est produit BIO101 est de préférence choisie parmi *Stemmacantha carthamoides* (aussi appelée *Leuzea carthamoides), Cyanotis arachnoidea* et *Cyanotis vaga.*

Les dérivés de phytoecdysones et notamment de 20-hydroxyecdysone sont obtenus par hémisynthèse et peuvent notamment être obtenus de la façon décrite dans la demande de brevet européenne n° EP 15732785.9.

Selon un mode de réalisation préféré, l'invention vise la composition pour son utilisation chez le mammifère dans le traitement d'une altération de la fonction respiratoire, plus particulièrement une altération de la fonction respiratoire résultant d'une pathologie neuromusculaire acquise ou génétique, par exemple une pathologie neuromusculaire des motoneurones et/ou de la jonction neuromusculaire et/ou du muscle strié squelettique.

Selon un mode de réalisation particulier, l'invention vise la composition pour son utilisation chez le mammifère dans le traitement d'une altération de la fonction respiratoire liée à une altération du muscle strié et/ou du muscle lisse.

Dans un mode de réalisation particulier, l'invention vise la composition pour son utilisation chez le mammifère dans le traitement d'une altération de la fonction respiratoire causée au moins en partie par une modification du muscle lisse. Selon un mode de réalisation particulier, l'invention vise la composition pour son utilisation chez le mammifère dans le traitement d'une altération de la fonction respiratoire liée à une hyper réactivité bronchique.

Dans un mode de réalisation, l'invention vise la composition pour son utilisation chez le mammifère dans le traitement d'une altération de la fonction respiratoire dans laquelle l'hyper réactivité bronchique est associée à la fonction du muscle lisse bronchique.

Dans un mode de réalisation, l'invention vise la composition pour son utilisation chez le mammifère dans le traitement d'une altération de la fonction respiratoire liée à une affection d'au moins un des paramètres respiratoires choisi parmi la valeur de la Penh, le pic maximal d'inspiration, le pic maximal d'expiration, le temps de relaxation, et la fréquence respiratoire. La composition va avantageusement réduire l'affection de ces paramètres respiratoires.

Dans un mode de réalisation, l'invention vise la composition comprenant pour son utilisation chez le mammifère dans le traitement d'une altération de la fonction respiratoire liée à une affection d'au moins un des paramètres mécaniques du tissu pulmonaire. Lesdits paramètres mécaniques du tissu pulmonaire sont l'élastance, la compliance et la résistance pulmonaire.

Dans un mode de réalisation particulier, l'invention vise la composition pour son utilisation chez le mammifère dans le traitement d'une altération de la fonction respiratoire liée à une diminution de la compliance pulmonaire et/ou une augmentation de la résistance pulmonaire et/ou une diminution de l'élastance pulmonaire.

Dans un mode de réalisation particulier, l'invention vise la composition pour son utilisation chez le mammifère dans le traitement d'une pathologie dans laquelle l'altération de la fonction respiratoire est liée à la rétractation et l'affaissement des alvéoles pulmonaires et/ou à l'apparition de fibrose.

Dans un mode de réalisation particulier, les phytoecdysones sont administrées à une dose comprise entre 3 et 15 milligrammes par kilogramme par jour chez l'humain. On entend ici par phytoecdysone, aussi bien les phytoecdysones de manière générale que leurs dérivés, la 20-hydroxyecdysone (notamment sous forme d'extrait) et ses dérivés.

De préférence, les phytoecdysones sont administrées à une dose de 200 à 1000 mg/jour, en une ou plusieurs prises, chez un humain adulte, et une dose de 5 à 350 mg/jour, en une ou plusieurs prises, chez l'humain enfant ou nourrisson. On entend ici par phytoecdysone, aussi bien les phytoecdysones de manière générale que leurs dérivés, la 20-hydroxyecdysone (notamment sous forme d'extrait) et ses dérivés.

Dans des modes de réalisation la composition comporte au moins un composé considéré comme un dérivé de phytoecdysone, ledit au moins un composé étant de formule générale (I) :

[Chem. 1]

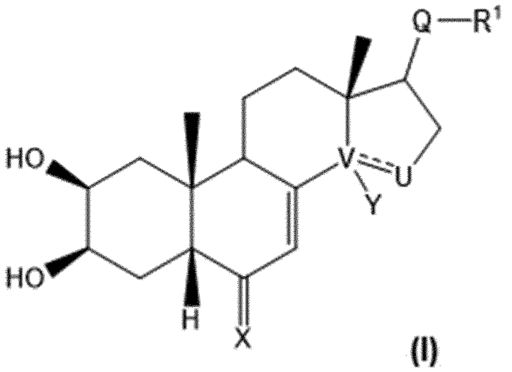

(I)

dans laquelle :

**V-U** est une liaison simple carbone-carbone et **Y** est un groupement hydroxyle:
**X** est un oxygène,
**Q** est un groupement carbonyle ;
**R**[1] est choisi parmi : un groupement $(C_1-C_6)$**W**$(C_1-C_6)$ ; un groupement $(C_1-C_6)$**W**$(C_1-C_6)$**W**$(C_1-C_6)$ ; un groupement $(C_1-C_6)$**W**$(C_1-C_6)CO_2(C_1-C_6)$; un groupement $(C_1-C_6)$**A**, **A** représentant un hétérocycle éventuellement substitué par un groupement de type OH, OMe, $(C_1-C_6)$, $N(C_1-C_6)$, $CO_2(C_1-C_6)$ ; un groupement $CH_2Br$ ;
**W** étant un hétéroatome choisi parmi N, O et S, de préférence O et encore plus préférentiellement S.

Dans le cadre de la présente invention on entend par « $(C_1-C_6)$ », tout groupe alkyle de 1 à 6 atomes de carbones, linéaire ou ramifié, en particulier, les groupes méthyle, éthyle, n-propyle, iso-propyle, n-butyle, isobutyle, sec-butyle, t-butyle, n-pentyle, n-hexyle. Avantageusement il s'agit d'un groupe méthyle, éthyle, iso-propyle ou t-butyle, en particulier d'un groupe méthyle ou éthyle, plus particulièrement d'un groupe méthyle.

Dans un mode de réalisation préféré, dans la formule (I) :

**Y** est un groupement hydroxyle ;
**R**[1] est choisi parmi : un groupement $(C_1-C_6)$**W**$(C_1-C_6)$ ; un groupement $(C_1-C_6)$**W**$(C_1-C_6)$**W**$(C_1-C_6)$ ; un groupement $(C_1-C_6)$**W**$(C_1-C_6)CO_2(C_1-C_6)$; un groupement $(C_1-C_6)$**A**, **A** représentant un hétérocycle éventuellement substitué par un groupement de type OH, OMe, $(C_1-C_6)$, $N(C_1-C_6)$, $CO_2(C_1-C6)$ ;
**W** étant un hétéroatome choisi parmi N, O et S, de préférence O et de préférence encore S.

Dans des modes de réalisation la composition comporte au moins un composé choisi parmi les composés suivants :

**n° 1 :** (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one,
**n° 2 :** (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one;
**n° 3 :** (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
**n° 4 :** (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
**n° 5** : (2S,3R,5R,10R,13R,14S,17S)-17-[2-(3-diméthylaminopropyl (méthyl)amino)acétyl]-2,3,14-trihydroxy-10,13-diméthyl-2,3,4,5,9,11,12,15,16,17-décahydro-1H-cyclopenta[a]phénanthrèn-6-one;
**n° 6 :** 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
**n° 7 :** (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
**n° 8** : (2S,3R,5R,10R,13R,14S,17S)-2,3,14-trihydroxy-17-[2-(2-hydroxyéthyl sulfanyl)acétyl]-10,13-diméthyl-2,3,4,5,9,11,12,15,16,17-décahydro-1H cyclopenta[a]phénanthrèn-6-one.

Dans des modes de réalisation la composition comporte au moins un composé considéré comme un dérivé de phytoecdysone, ledit au moins un composé étant de formule générale (II) :

[Chem. 2]

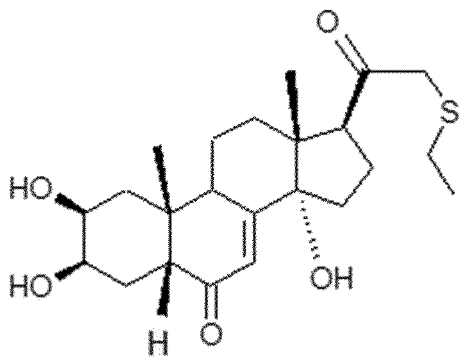

(II)

Le composé de formule (II) est par la suite appelé BIO103.

Dans des modes de réalisation la composition

est incorporée à une formulation pharmaceutique acceptable pouvant être administrée par voie orale.

Dans le cadre de la présente invention on entend par « pharmaceutique acceptable » ce qui est utile dans la préparation d'une composition pharmaceutique qui est généralement sûre, non toxique et qui est acceptable pour une utilisation vétérinaire de même que pharmaceutique humaine.

## Brève description des figures

L'invention sera mieux comprise à la lecture de la description suivante, donnée à titre d'exemple nullement limitatif, et faite en se référant aux figures qui représentent :

La figure 1A représente la courbe de la valeur de la Penh avant le début de l'étude, de souris contrôles saines C57Black10 (n=12, ronds blancs) et de souris mdx (n=23, carrés blancs) avant de recevoir le traitement (J0 : jour zéro), mesurée par pléthysmographie, en réponse à des doses croissantes de métacholine avec **p<0,001 et ***p<0,0001. Dans la suite de la description, n correspond à la taille de l'échantillon et p correspond à la « p-valeur » utilisée pour quantifier la significativité statistique d'un résultat avec * : p<0,05 ; ** : p<0,001 ; ***p<0,0001 et ns : non significatif.

La figure 1B représente la courbe du pic maximal d'inspiration avant le début de l'étude, de souris contrôles saines C57Black10 (n=12, ronds blancs) et de souris mdx (n=23, carrés blancs) avant de recevoir le traitement (J0 : jour zéro), mesuré par pléthysmographie, en réponse à des doses croissantes de métacholine avec *p<0,05, **p<0,001 ;

La figure 1C représente la courbe du pic maximal d'expiration avant le début de l'étude, de souris contrôles saines C57Black10 (n=12, ronds blancs) et de souris mdx (n=23, carrés blancs) avant de recevoir le traitement (J0 : jour zéro), mesuré par pléthysmographie, en réponse à des doses croissantes de métacholine avec **p<0,001 ;

La figure 1D représente la courbe du temps de relaxation avant le début de l'étude, de souris contrôles saines C57Black10 (n=12, ronds blancs) et de souris mdx (n=23, carrés blancs) avant de recevoir le traitement (J0 : jour zéro), mesuré par pléthysmographie, en réponse à des doses croissantes de métacholine avec **p<0,001 et ***p<0,0001 ;

La figure 1E représente la courbe de la fréquence respiratoire avant le début de l'étude, de souris contrôles saines C57Black10 (n=12, ronds blancs) et de souris mdx (n=23, carrés blancs) avant de recevoir le traitement (J0 : jour zéro), mesurée par pléthysmographie, en réponse à des doses croissantes de métacholine avec *p<0,05, et ***p<0,0001 ;

La figure 2A représente la courbe de la valeur de la Penh avant le début de l'étude (J0), mesurée par pléthysmographie, en réponse à des doses croissantes de métacholine avec **p<0,001 et ***p<0,0001, après la randomisation des animaux dans les trois différents groupes : souris contrôles saines C57Black10 (n=12, ronds blancs), souris mdx qui ne recevront pas de traitement (n=11, carrés blancs, groupe « mdx ») et de souris mdx qui seront traitées avec BIO101 (n=12, carrés noirs, groupe « mdx BIO101 ») ;

La figure 2B représente la courbe du pic maximal d'inspiration avant le début de l'étude (J0), mesuré par pléthysmographie, en réponse à des doses croissantes de métacholine, après la randomisation des animaux dans les trois différents groupes : souris contrôles saines C57Black10 (n=12, ronds blancs), souris mdx qui ne recevront pas de traitement (n=11, carrés blancs, groupe « mdx ») et de souris mdx qui seront traitées avec BIO101 (n=12, carrés noirs, groupe « mdx BIO101 ») ; La figure 2C représente la courbe du pic maximal d'expiration avant le début de l'étude (J0), mesuré par pléthysmographie, en réponse à des doses croissantes de métacholine, après la randomisation des animaux dans les trois différents groupes : souris contrôles saines C57Black10 (n=12, ronds blancs), souris mdx qui ne recevront pas de traitement (n=11, carrés blancs, groupe « mdx ») et de souris mdx qui seront traitées avec BIO101 (n=12, carrés noirs, groupe « mdx BIO101 ») ;

La figure 2D représente la courbe du temps de relaxation avant le début de l'étude (J0), mesuré par pléthysmographie, en réponse à des doses croissantes de métacholine, après la randomisation des animaux dans les trois différents groupes : souris contrôles saines C57Black10 (n=12, ronds blancs), souris mdx qui ne recevront pas de traitement (n=11, carrés blancs, groupe « mdx ») et de souris mdx qui seront traitées avec BIO101 (n=12, carrés noirs, groupe « mdx BIO101 ») ;

La figure 2E représente la courbe de la fréquence respiratoire avant le début de l'étude (J0), mesurée par pléthysmographie, en réponse à des doses croissantes de métacholine, après la randomisation des animaux dans les trois différents groupes : souris contrôles saines C57Black10 (n=12, ronds blancs), souris mdx qui ne recevront pas de traitement (n=11, carrés blancs, groupe « mdx ») et de souris mdx qui seront traitées avec BIO101 (n=12, carrés noirs, groupe « mdx BIO101 ») ;

La figure 3A représente la courbe de la valeur de Penh après 30 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesurée par pléthysmographie, en réponse à des doses croissantes de métacholine avec **p<0,001 ;

La figure 3B représente la courbe du pic maximal

d'inspiration après 30 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesuré par pléthysmographie, en réponse à des doses croissantes de métacholine avec *p<0,05, **p<0,001 ;

La figure 3C représente la courbe du pic maximal d'expiration après 30 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesuré par pléthysmographie, en réponse à des doses croissantes de métacholine avec *p<0,05 ;

La figure 3D représente la courbe du temps de relaxation après 30 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesuré par pléthysmographie, en réponse à des doses croissantes de métacholine avec *p<0,05 ;

La figure 3E représente la courbe de la fréquence respiratoire après 30 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesurée par pléthysmographie, en réponse à des doses croissantes de métacholine ;

La figure 4A représente la courbe de la valeur Penh après 60 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesurée par pléthysmographie, en réponse à des doses croissantes de métacholine avec ***p<0,0001 ;

La figure 4B représente la courbe du pic maximal d'inspiration après 60 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesuré par pléthysmographie, en réponse à des doses croissantes de métacholine avec *p<0,05 ;

La figure 4C représente la courbe du pic maximal d'expiration après 60 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesuré par pléthysmographie, en réponse à des doses croissantes de métacholine ;

La figure 4D représente la courbe du temps de relaxation après 60 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesuré par pléthysmographie, en réponse à des doses croissantes de métacholine ;

La figure 4E représente la courbe de la fréquence respiratoire après 60 jours de traitement, de souris contrôles saines C57Black10 (n=12, ronds blancs), de souris mdx non traitées (n=11, carrés blancs, groupe « mdx ») et de souris mdx traitées avec BIO101 (n=12, carrés noirs, groupe « mdx BIO101 »), mesurée par pléthysmographie, en réponse à des doses croissantes de métacholine ;

La figure 5A représente la courbe de l'évolution de la valeur de Penh mesurée par pléthysmographie, de souris saines C57Black10 en début d'étude (n=12, ronds noirs, J0) et à la fin de l'étude (n=12, ronds blancs, J60), soumises à des doses croissantes de métacholine ;

La figure 5B représente la courbe de l'évolution de la valeur de Penh mesurée par pléthysmographie, de souris mdx non traitées en début d'étude (n=23, ronds noirs, J0) et à la fin de l'étude (n=12, ronds blancs, J60), soumises à des doses croissantes de métacholine avec ***p<0,0001 ;

La figure 6A représente les valeurs de Penh au début de l'étude (J0) de souris saines C57Black10 (n=12) et de souris mdx non traitées (n=23) mesurées par pléthysmographie à une dose de métacholine de 40 mg/ml avec ***p<0,0001 ;

La figure 6B représente les valeurs de Penh après 60 jours d'étude (J60) de souris saines C57Black10 (n=12), de souris mdx non traitées (n=12) et de souris mdx traitées avec BIO101 (n=12) mesurées par pléthysmographie à une dose de métacholine de 40 mg/mL avec ***p<0,0001 ;

La figure 7A représente l'évolution de la résistance pulmonaire mesurées par ventilateur à piston (FlexiVent™) à des doses croissantes de métacholine, chez des souris saines C57Black10 (n=10), de souris mdx non traitées (n=10) et de souris mdx traitées avec BIO101 (n=10) avec **p<0,001 et ***p<0,0001 ;

La figure 7B représente l'évolution de la compliance pulmonaire mesurées par ventilateur à piston à des doses croissantes de métacholine, chez des souris saines C57Black10 (n=10), de souris mdx non traitées (n=10) et de souris mdx traitées avec BIO101 (n=10) avec ***p<0,0001 ;

La figure 7C représente l'évolution de la élastance pulmonaire mesurées par ventilateur à piston à des doses croissantes de métacholine, chez des souris saines C57Black10 (n=10), de souris mdx non traitées (n=10) et de souris mdx traitées avec BIO101 (n=10) avec ***p<0,0001 ;

La figure 8A représente les valeurs de résistance

pulmonaire après 60 jours d'étude de souris saines C57Black10 (n=10), de souris mdx non traitées (n=10) et de souris mdx traitées avec BIO101 (n=10) mesurées par ventilateur à piston à une dose de métacholine de 20 mg/mL avec ***p<0,0001 ;

La figure 8B représente les valeurs de compliance pulmonaire après 60 jours d'étude de souris saines C57Black10 (n=10), de souris mdx non traitées (n=10) et de souris mdx traitées avec BIO101 (n=10) mesurées par ventilateur à piston à une dose de métacholine de 20 mg/mL avec ***p<0,0001 ;

La figure 8C représente les valeurs d'élastance pulmonaire après 60 jours d'étude de souris saines C57Black10 (n=10), de souris mdx non traitées (n=10) et de souris mdx traitées avec BIO101 (n=10) mesurées par ventilateur à piston à une dose de métacholine de 20 mg/mL avec *p<0,05 et **p<0,001.

## Description des modes de réalisation

L'invention sera décrite ci-après dans le contexte particulier de certains de ses domaines d'application préférés, non limitatif.

1. Procédé de purification de BIO101

BIO101 est préparé à partir de 20-hydroxyecdysone pure à de l'ordre de 90%, selon les étapes suivantes :

i) dissolution à chaud de 20-hydroxyecdysone pure à de l'ordre de 90% dans du méthanol, filtration et concentration partielle,
ii) ajout de 3 volumes d'acétone,
iii) refroidissement à une température comprise entre 0 et 5°C, sous agitation,
iv) filtration du précipité obtenu,
v) rinçages successifs avec de l'acétone et de l'eau, et
vi) séchage.

Cette purification met en jeu un processus de recristallisation approprié à cette molécule et apte à être réalisé à l'échelle industrielle.

La filtration de l'étape i) est réalisée grâce à un filtre à particules de 0,2 $\mu$m.

La concentration partielle de l'étape i) est avantageusement effectuée par distillation sous vide, à une température de l'ordre de 50°C, en présence de MeOH.

L'étape vi) de séchage est effectuée sous vide à une température de l'ordre de 50°C.

2. Activité biologique de BIO101

Des souris mâles C57BL/10ScSnJ (souris saines dénommées « C57Black10 » dans les figures) et C57BL/10ScSn-Dmdmdx/J (modèle murin de la dystrophie musculaire de Duchenne, dénommées « mdx » dans les figures) âgées de douze semaines, ont été utilisées. Les souris ont été réparties en trois groupes de 12 souris chacun : un groupe non traité de souris contrôle saines C57Black10, un groupe de souris mdx non traitées (mdx) et un groupe de souris traitées à une dose de 50 mg/kg/jour par voie orale, de manière chronique, dans l'eau de boisson.

La fonction respiratoire de toutes les souris a été évaluée par pléthysmographie sur corps entier avant le début de l'étude, (J0) puis 30 jours (J30) et 60 jours (J60) après le début du traitement. Après deux mois de traitement, la fonction respiratoire a été évaluée de manière invasive, juste avant le sacrifice de l'animal, par ventilateur à piston commandé par unité centrale de commande (communément appelé « ordinateur »), ledit ventilateur utilisant la technique de l'oscillation forcée comme l'appareil connu sous le nom FlexiVent™, afin de déterminer plus directement les paramètres mécaniques du système respiratoire.

*a. Analyse par pléthysmographie des effets de 810101 sur la fonction respiratoire*

Afin de suivre précisément la modification de la fonction respiratoire au cours du traitement, des analyses de pléthysmographie (Emka Technologies, Paris, France) sur corps entier ont été réalisées sur des souris C57Black10 saines contrôles, sur des souris mdx non traitées et sur des souris mdx ayant été traitées avec BIO101. Les avantages de cette technique résident dans le fait qu'elle permet de réaliser un suivi sur un animal vigile, libre de ses mouvements dans une enceinte hermétique et ce, de manière non invasive. Par conséquent, le stress dû aux manipulations des animaux est diminué et il est possible de répéter les mesures au cours de périodes prolongées. La pléthysmographie barométrique est donc très utilisée pour mesurer la réactivité bronchique chez les petits animaux (Chong et al., 1998 ; Djuric et al., 1998 ; Hoffman et al., 1999).

Les variations de pressions mesurées par rapport à une chambre de référence permettent de définir différents paramètres respiratoires tels que les pics et les temps de pression inspiratoire et expiratoire ainsi qu'une grandeur sans unité appelée Penh (*enhanced Pause*) qui permet d'évaluer la broncho-constriction. En effet, la valeur de la Penh, calculée à partir du signal de pression dans la chambre (Pb), est un index important à obtenir, car les variations de la Penh évoluent en parallèle avec celles de la résistance respiratoire et elle représente donc un paramètre prédictif des changements des propriétés résistives du système respiratoire (Hamelmann et al., 1997 ; Bergren, 2001 ; Onclinx et al., 2003). Les valeurs suivantes ont été calculées à partir de la Pb filtrée : la variation maximale de Pb pendant l'expiration (*PEP : Peak expiratory Pressure*), la modification maximale de Pb pendant l'inspiration (PIP : *Peak Inspiratory Pressure*) et l'intervalle de temps (TR). Ensuite, la Penh a été calculée comme suit :

[Math. 1] (PIP/PEP) x Pause

où

[Math. 2] Pause = (TE-TR) / TE

TE étant le temps expiratoire (Adler et al., 2004).

De plus, ont été également mesurés et représentés le pic maximal d'inspiration et le pic maximal d'expiration (PIF : *Peak Inspiratory Flow* et PEF : *Peak Expiratory Flow),* le temps de relaxation (RT) et la fréquence respiratoire (BF).

La valeur de la Penh a été mesurée avant le début du traitement (J0), 30 jours après le début du traitement (J30) et 60 jours après le traitement (J60).

Dans un premier temps, la cohorte entière des souris mdx (n=24) a été comparée aux souris saines C57Black10 (n=12), avant de recevoir le traitement. Des souris vigiles ont été exposées à des doses croissantes d'aérosols de métacholine générés par un nébuliseur contenant 0 à 40 mg/mL de métacholine dans du PBS. De manière attendue et déjà démontrée, les souris mdx présentent une altération de leur fonction respiratoire (Huang et al., 2011 ; Gosselin et al., 2003 ; Gayraud et al., 2007 ; Ishizaki et al., 2008) avec une augmentation significative de la Penh en réponse à la métacholine (p<0,001 et p<0.0001) (**Figure 1A)** associée à un pic maximal d'inspiration et à un pic maximal d'expiration significativement inférieurs (p<0,05 et p<0.001) (**Figures 1B et 1C)** en comparaison avec des souris saines, en particulier avant ou à de faible dose de broncho-constricteur. Le temps de relaxation (RT) est également très significativement supérieur chez des souris mdx en comparaison à des souris contrôles (p<0.001 et p<0.0001) (**Figure 1D**). Les valeurs de temps inspiratoire (TI) et de temps expiratoire (TE) restent comparables entre les groupes à chaque moment de la mesure (données non présentées). La fréquence de respiration des souris mdx est aussi diminuée par rapport à des souris saines (p<0,05 et p<0.0001) (**Figure 1E**).

Les souris mdx ont ensuite été réparties dans deux groupes différents : les souris mdx qui ne recevront aucun traitement (carrée blancs) et les souris mdx qui recevront la molécule BIO101 (carrés noirs). Comme le montre les **Figure 2A, 2B, 2C, 2D** et **2E,** ces deux groupes présentent les mêmes capacités de leur fonction respiratoire sans différence significative entre les différents paramètres respiratoires mesurés, ce qui montre qu'avant le traitement, toutes les souris mdx non traitées possèdent le même profil respiratoire.

Après 30 jours de traitement, les souris mdx traitées avec BIO101 présentent une diminution significative de Penh (p<0,001) par rapport aux souris mdx à J0, avec une courbe de la Penh en réponse à la métacholine comparable à celle des souris saines C57Black10 **(Figure 3A).** La diminution de la Penh est associée à une augmentation du pic maximal d'inspiration, en particulier à l'état d'équilibre ou à une faible dose de métacholine (p<0.05) **(Figure 3C).** De plus, on observe une nette amélioration du temps de relaxation chez les souris mdx traitées avec BIO101 par rapport aux souris mdx non traitées (p<0.05) **(Figure 3D).** En revanche, le pic maximal d'inspiration et la fréquence respiratoire restent inchangés par rapport à celui mesuré à J0 **(Figures 3B et 3E).**

Après 60 jours de traitement, BIO101 conserve son effet bénéfique sur les fonctions respiratoires des souris mdx. En effets, les résultats montrent la confirmation de ce qui a été observé après 30 jours de traitement. Les souris mdx traitées avec BIO101 montrent une diminution significative de la valeur de la Penh par rapport aux souris mdx non traitées (p<0,0001) et le profil des variations de la valeur de la Penh est similaire à celui des témoins, quelle que soit la dose de métacholine **(Figure 4A).** On observe une amélioration significative du pic maximal d'inspiration (p<0.05) conjointement avec une tendance à l'amélioration du temps de relaxation **(Figures 4B et 4D).** En revanche, le pic maximal d'expiration et la fréquence respiratoire restent inchangés **(Figures 4B et 4E).**

Pour évaluer de manière longitudinale l'altération de la fonction respiratoire chez les souris mdx, la variation de la valeur de la Penh a été comparée entre J0 et J60 chez des souris saines contrôles ainsi qu'entre J0 et J60 chez des souris mdx non traitées. De manière attendue, la Penh n'a pas changé entre le J0 et J60 chez les souris témoins (p=ns) **(Figure 5A).** En revanche, la fonction respiratoire s'est significativement dégradée chez les souris mdx à J60 par rapport à J0, comme le montre l'augmentation de Penh en réponse à la métacholine (p<0,0001) **(Figure 5B).**

A une dose de métacholine de 40mg/mL, la valeur moyenne de la Penh chez des souris saines contrôles (n=12) est significativement inférieure à celle retrouvée chez des souris mdx (n=23) avant traitement (respectivement Penh=0,72 et 1,42 avec p<0,0001) **(Figure 6A**). A J60, cette valeur de la Penh est d'autant plus augmentée chez des souris mdx non traitées (n=12) par rapport à des souris contrôles saines (n=12) avec des valeurs respectivement de 4 et 1,04 (p<0,0001) **(Figure 6B).** De manière intéressante, le traitement par BIO101 diminue significativement cette valeur, avec une valeur de Penh égale à 1,87 (p<0,0001) comparé aux souris mdx non traitées **(Figure 6B).** Le traitement diminue la valeur de la Penh des souris mdx à un niveau non significativement différent de celle des souris saines (Penh= 1,04 chez des souris C57Black10 versus 1,87 chez des souris mdx avec p<0.05).

*b. Analyse par ventilateur à piston des effets de 810101 sur la résistance, la compliance et l'élastance pulmonaires*

Afin de déterminer plus directement les paramètres mécaniques du système respiratoire et l'impact d'un traitement chronique pendant deux mois par BIO101 (J60) sur ces paramètres, la résistance pulmonaire dynamique a été mesurée à l'aide d'un système de ventilateur à piston tel que décrit précédemment, en réponse à des doses croissantes de métacholine.

Les souris ont été anesthésiées et connectées via une canule endotrachéale au système de ventilateur à piston. Après le début de la ventilation mécanique, chaque souris a reçu une injection intrapéritonéale de 0,1 mL d'une solution de bromure de rocuronium à 10 mg/mL. L'animal a été ventilé à une fréquence respiratoire de 150 respirations/minute et avec un volume courant de 10 mL/kg contre une pression expiratoire finale positive de 3 cm $H_2O$. Les mécanismes respiratoires ont été évalués à l'aide d'une manoeuvre d'oscillation forcée d'une durée de 1,2 secondes (2,5 Hz) et d'une manoeuvre d'oscillation forcée de 3 secondes à large bande contenant 13 fréquences primordiales comprises entre 1 et 20,5 Hz. La résistance du système respiratoire (R) a été calculée à l'aide d'une unité centrale de traitement de type microordinateur reliée au système de ventilateur à piston et comprenant notamment un logiciel configuré pour réaliser ledit calcul. Les deux manoeuvres sont exécutées toutes les 15 secondes en alternance après chaque nébulisation avec un aérosol de métacholine pour mesurer l'évolution dans le temps et la réponse de la bronchoconstriction induite par la métacholine. La résistance, la compliance et l'élastance pulmonaire ont ainsi pu être déterminées.

En adéquation avec les résultats obtenus en pléthysmographie, on observe que les souris mdx non traitées ont une résistance des voies respiratoires supérieure à celle des souris saines C57Black10 (p<0,001) **(Figure 7A).** Après 60 jours de traitement, en réponse à des doses croissantes de métacholine de 0 à 20 mg/mL, les souris mdx traitées avec BIO101 présentent une résistance des voies respiratoires significativement plus faible que les souris mdx non traitées (p<0,0001), comparable au niveau de résistance pulmonaire observé chez les souris saines contrôles **(Figure 8A).** De la même façon, le traitement chronique pendant deux mois avec BIO101 améliore significativement la compliance (p<0.0001) et l'élastance (p<0.0001) pulmonaire, deux paramètres qui sont altérée chez les souris mdx **(Figures 7B et 7C).** En effet, à la plus forte dose testée de métacholine (20 mg/mL), les souris mdx présentent une compliance pulmonaire significativement diminuée par rapport à des souris C57Black10 contrôles (respectivement 0,017 mL/cm$H_2O$ et 0,031 mL/cm$H_2O$ avec p<0,0001). Ce défaut de compliance pulmonaire est corrigé de manière significative avec le traitement par BIO101 des souris mdx (0,029 mL/cm$H_2O$ avec p<0,0001) **(Figure 8 B).** De façon identique, l'élastance pulmonaire est très diminuée chez des souris mdx par rapport à des souris C57Black10 contrôles (34,8 cm$H_2O$/mL versus 62,8 cmH20/mL, avec p<0,05) et le traitement par BIO101 permet de maintenir l'élastance pulmonaire similaire à celle de souris saines contrôle et significativement plus importante que des souris mdx non traitées (34,8 cm$H_2O$/mL versus 69,1 cm$H_2O$/mL avec p<0,001) **(Figure 8 C).**

3. <u>Conclusion</u>

Ces résultats démontrent que le traitement par BIO101 (50 mg/kg par jour dans l'eau de boisson) améliore la fonction respiratoire des souris mdx (un modèle animal de la dystrophie musculaire de Duchenne) et ce, de manière prolongée au cours du temps. Cet effet sur la fonction respiratoire est associé non seulement à des paramètres respiratoires (durée et fréquence inspiratoires et expiratoires), comme le démontrent les résultats de pléthysmographie et en particulier, les mesures de la Penh, mais démontrent également une amélioration de la structure des voies respiratoires profondes démontrée par les expériences utilisant le système de ventilateur à piston. En effet, les données de ce système démontrent significativement des effets bénéfiques du traitement par BIO101 sur les paramètres mécaniques respiratoires de résistance, de compliance et de l'élastance des poumons chez les souris mdx. Ces observations sont la conséquence d'une protection du traitement avec les phytoecdysones, notamment BIO101, de la dégradation des fonctions pulmonaires au cours du temps, dans un modèle murin de pathologie neuromusculaire.

De manière plus générale, il est à noter que les modes de mise en oeuvre et de réalisation de l'invention considérés ci-dessus ont été décrits à titre d'exemples non limitatifs et que d'autres variantes sont par conséquent envisageables.

**Références bibliographiques**

Adler A, Cieslewicz G, Irvin CG. Unrestrained plethysmography is an unreliable measure of airway responsiveness in BALB/c and C57BL/6 mice. J. Appl. Physiol. (1985). (2004); 97(1):286-92.

Baydur A, Gilgoff I, Prentice W, Carlson M, Fischer DA. Decline in respiratory function and expérience with long-term assisted ventilation in advanced Duchenne's muscular dystrophy. Chest (1990); 97:884-9.

Benditt JO and Boitano LJ. Pulmonary Issues in Patients with Chronic Neuromuscular Disease, Am. J. Respir. Crit. Care Med. (2013); 187 (10):1046-55.

Bergren DR. Chronic tobacco smoke exposure increases airway sensitivity to capsaicin in awake guinea pigs. j. Appl. Physiol. (2001); 90:695-704.

Birnkrant DJ, Panitch HB, Benditt JO, Boitano LJ, Carter ER, et al. American College of Chest Physicians consensus statement on the respiratory and related management of patients with Duchenne muscular dystrophy undergoing anesthesia or sedation. Chest (2007); 132:1977-86.
Chong BT, Agrawal DK, Romero FA, Townley RG. Measurement of bronchoconstriction using whole-body plethysmograph: comparison of freely moving versus restrained guinea pigs. J. Pharmacol. Toxicol. Methods (1998); 39:163-8.
Djuric VJ, Cox G, Overstreet DH, Smith I, Dragomir A, Steiner M. Genetically transmitted cholinergic hyperresponsiveness predisposes to experimental asthma. Brain Behav. Immun. (1998); 12:272-84.
Finder JD, Birnkrant D, Carl J, Farber HJ, Gozal D, et al. American Thoracic Society. Respiratory care of the patient with Duchenne muscular dystrophy: ATS consensus statement. Am. J. Respir. Crit. Care Med. (2004); 170:456-65.
Gayraud J, Matecki S, Hnia K, Mornet D, Prefaut C, et al. Ventilation during airbreathing and in response to hypercapnia in 5 and 16 month-old mdx and C57 mice. J. Muscle Res. Cell Motil. (2007); 28(1):29-37.
Gosselin LE, Barkley JE, Spencer MJ, McCormick KM, Farkas GA. Ventilatory dysfunction in mdx mice: impact of tumor necrosis factor-alpha deletion. Muscle Nerve (2003); 28:336-43.
Hamelmann E, Schwarze J, Takeda K, Oshiba A, Larsen GL, Irvin CG, Gelfand EW. Noninvasive measurement of airway responsiveness in allergic mice using barometric plethysmography. Am. J. Respir. Crit. Care Med. (1997); 156:766-75.
Hoffman AM, Dhupa N, Cimetti L. Airway reactivity measured by barometric whole-body plethysmography in healthy cats. Am. J. Vet. Res., (1999); 60:1487-92.
Huang P, Cheng G, Lu H, Aronica M, Ransohoff RM, Zhou L. Impaired respiratory function in mdx and mdx/utrn(+/-) mice. Muscle Nerve (2011); 43(2):263-7.
Inkley SR, Oldenburg FC, Vignos PJ Jr. Pulmonary function in Duchenne muscular dystrophy related to stage of disease. Am. J. Med. (1974); 56:297-306.
Ishizaki M, Suga T, Kimura E, Shiota T, Kawano R, et al. Mdx respiratory impairment following fibrosis of the diaphragm. Neuromuscul. Disord. (2008); 18(4):342-8.
Lo Mauro A and Aliverti A. Physiology of respiratory disturbances in muscular dystrophies. Breathe (2016); 12(4):318-27.
Matecki S, Rivier F, Hugon G, Koechlin C, et al. The effect of respiratory muscle training with CO2 breathing on cellular adaptation of mdx mouse diaphragm. Neuromuscul. Disord. (2005); 15:427-36.
Mayer OH, Finkel RS, Rummey C, Benton MJ, Glanzman AM, et al. Characterization of Pulmonary Function in Duchenne Muscular Dystrophy, Pediatr. Pulmonol. (2015); 50:487-94.
McKim DA, Road J, Avendano M, Abdool S, Cote F, et al. Canadian Thoracic Society Home Mechanical Ventilation Committee. Home mechanical ventilation: a Canadian Thoracic Society clinical practice guideline. Can. Respir. J. (2011); 18:197-215.
Miller RG, Jackson CE, Kasarskis EJ, England JD, Forshew D, et al. Quality Standards Subcommittee of the American Academy of Neurology. Practice parameter update: the care of the patient with amyotrophic lateral sclerosis: drug, nutritional, and respiratory thérapies (an evidence-based review): report of the Quality Standards Subcommittee of the American Academy of Neurology. Neurology (2009); 73:1218-26.
Mosqueira M, Baby SM, Lahiri S, Khurana TS. Ventilatory chemosensory drive is blunted in the mdx mouse model of Duchenne Muscular Dystrophy (DMD). PLoS One. (2013); 8(7):e69567
Onclinx C. Relation entre la résistance pulmonaire totale et la Penh en fonction de la localisation anatomique de l'obstruction des voies aériennes (mémoire-diplôme d'étude approfondie). Université de Liège, Faculté de médecine vétérinaire : Liège, (2003), 24p.
Smith PE, Calverley PM, Edwards RH, Evans GA, Campbell EJ. Practical problems in the respiratory care of patients with muscular dystrophy. N. Engl. J. Med. (1987); 316:1197-205.
Vianello A, Bevilacqua M, Salvador V, Cardaioli C, Vincenti E. Long-term nasal intermittent positive pressure ventilation in advanced Duchenne's muscular dystrophy. Chest (1994); 105:445-8.

## Revendications

1. Composition comprenant au moins une phytoecdysone et/ou au moins un dérivé hémi-synthétique de phytoecdysone pour son utilisation chez le mammifère dans le traitement d'une altération de la fonction respiratoire résultant d'une pathologie neuromusculaire acquise ou génétique, ou d'une altération de la fonction respiratoire liée à une hyper réactivité bronchique, ledit au moins un dérivé hémi-synthétique de phytoecdysone étant choisi parmi :

   - un composé de formule générale (I) :

[Chem. 1]

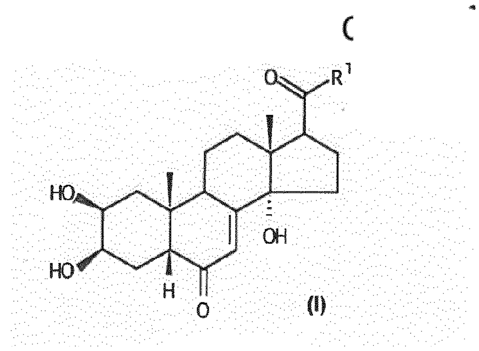

(I)

dans laquelle :

**$R^1$** est choisi parmi : un groupement $(C_1-C_6)$**W**$(C_1-C_6)$ ; un groupement $(C_1-C_6)$**W**$(C_1-C_6)$**W**$(C_1-C_6)$ ; un groupement $(C_1-C_6)$**W**$(C_1-C_6)CO_2(C_1-C_6)$; un groupement $(C_1-C_6)$**A**, **A** représentant un hétérocycle éventuellement substitué par un groupement de type OH, OMe, $(C_1-C_6)$, $N(C_1-C_6)$, $CO_2(C_1-C_6)$ ; un groupement $CH_2Br$ ;
**W** étant un hétéroatome choisi parmi N, O et S, de préférence O et encore plus préférentiellement S.

- le composé de formule (II) :

[Chem. 2]

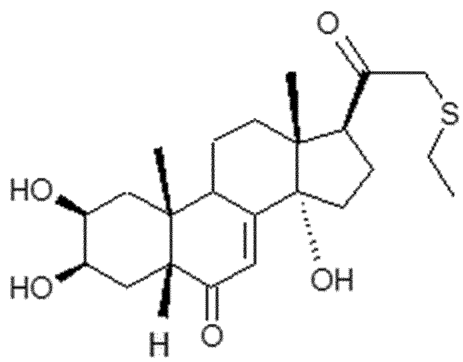

(II)

2. Composition pour son utilisation selon la revendication 1, comportant de la 20-hydroxyecdysone.

3. Composition pour son utilisation selon la revendication 2, dans laquelle la 20-hydroxyecdysone est sous forme d'extrait de plante ou d'une partie de plante, ladite plante étant choisie parmi les végétaux contenant au moins 0,5% de 20-hydroxyecdysone en poids sec dudit végétal, ledit extrait comportant au moins 95%, et de préférence au moins 97%, de 20-hydroxyecdysone.

4. Composition pour son utilisation selon la revendication 3, comportant de façon remarquable entre 0 et 0,05 %, en poids sec de l'extrait, d'impuretés susceptibles d'affecter l'innocuité, la disponibilité ou l'efficacité d'une application pharmaceutique dudit extrait.

5. Composition pour son utilisation selon l'une quelconque des revendications 3 à 4, dans laquelle la plante est choisie parmi *Stemmacantha carthamoides, Cyanotis arachnoidea* et *Cyanotis vaga.*

6. Composition pour son utilisation selon l'une quelconque des revendications 1 à 5, dans laquelle l'altération de la fonction respiratoire résulte d'une pathologie neuromusculaire des motoneurones et/ou de la jonction neuromusculaire et/ou du muscle strié squelettique.

7. Composition pour son utilisation selon l'une quelconque des revendications 1 et 6, dans laquelle l'altération de la fonction respiratoire est liée à une altération du muscle strié et/ou du muscle lisse.

8. Composition pour son utilisation selon l'une quelconque des revendications 1 à 5, dans laquelle l'hyper réactivité bronchique est associée à la fonction du muscle lisse bronchique.

9. Composition pour son utilisation selon l'une quelconque des revendications 1 à 8, dans laquelle l'altération de la fonction respiratoire est liée à une affection d'au moins un des paramètres respiratoires choisi parmi la valeur de Penh, le pic maximal d'inspiration, le pic maximal d'expiration, le temps de relaxation, et la fréquence respiratoire.

10. Composition pour son utilisation selon l'une quelconque des revendications 1 à 9, dans laquelle l'altération de la fonction respiratoire est liée à une affection d'au moins un des paramètres mécaniques du tissu pulmonaire.

11. Composition pour son utilisation selon la revendication 10, dans laquelle l'altération de la fonction respiratoire est liée à une diminution de la compliance pulmonaire et/ou une augmentation de la résistance pulmonaire et/ou une diminution de l'élastance pulmonaire.

12. Composition pour son utilisation selon l'une quelconque des revendications 1 à 11, dans laquelle les phytoecdysones sont administrées à une dose comprise entre 3 et 15 milligrammes par kilogramme par jour chez l'humain.

13. Composition pour son utilisation selon l'une quelconque des revendications 1 à 12, dans laquelle les phytoecdysones sont administrées à une dose de 200 à 1000 mg/jour, en une ou plusieurs prises, chez un humain adulte, et une dose de 5 à 350 mg/jour, en une ou plusieurs prises, chez l'humain enfant ou le nourrisson.

14. Composition pour son utilisation selon l'une quelconque des revendications 1 à 13, dans laquelle ledit au moins un composé de formule générale (I) est choisi parmi :

- **n°1** : (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one;
- **n°2** : (2S,3R,5R,10R,13R,14S,17S)-2,3,14-trihydroxy-17-[2-(3-hydroxypyrrolidin-1 -yl)acétyl]-10,13-diméthyl-2,3,4,5,9,11,12,15,16,17-décahydro-1H-cyclopenta[a]phénanthrèn-6-one;
- **n°3** : (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
- **n°4** : (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
- **n°5 :** (2S,3R,5R,10R,13R,14S,17S)-17-[2-(3-diméthylaminopropyl(méthyl)amino)acétyl]-2,3,14-trihydroxy-10,13-diméthyl-2,3,4,5,9,11,12,15,16,17-décahydro-1H-cyclopenta[a]phénanthrèn-6-one;
- **n°6** : 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
- **n°7** : (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
- **n°8** : (2S,3R,5R,10R,13R,14S,17S)-2,3,14-trihydroxy-17-[2-(2-hydroxyéthylsulfanyl)acétyl]-10,13-diméthyl-2,3,4,5,9,11,12,15,16,17-décahydro-1H-cyclopenta[a]phénanthrèn-6-one.

## Patentansprüche

**1.** Zusammensetzung, die mindestens ein Phytoecdyson und/oder mindestens ein halbsynthetisches Derivat von Phytoecdyson umfasst, zu ihrer Verwendung beim Säugetier in der Behandlung einer Veränderung der Atemfunktion, die auf eine erworbene oder genetisch bedingte neuromuskuläre Erkrankung zurückgeht, oder einer Veränderung der Atemfunktion, die mit einer Hyperreaktivität der Bronchien zusammenhängt, wobei das mindestens eine halbsynthetische Phytoecdyson-Derivat ausgewählt ist aus:

- einer Verbindung der allgemeinen Formel (I):

[Chem. 1]

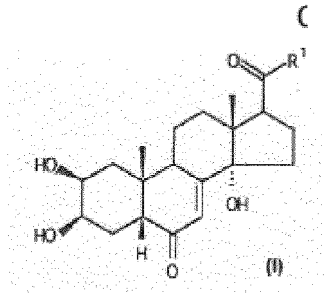

(I)

wobei:

**R¹** ausgewählt ist aus: einer $(C_1-C_6)$**W**$(C_1-C_6)$-Gruppe; einer $(C_1-C_6)$**W**$(C_1-C_6)$**W**$(C_1-C_6)$-Gruppe; einer $(C_1-C_6)$**W**$(C_1-C_6)CO_2(C_1-C_6)$-Gruppe; einer $(C_1-C_6)$**A**-Gruppe, wobei **A** einen Heterocyclus darstellt, der gegebenenfalls durch eine Gruppe vom Typ OH, OMe, $(C_1-C_6)$, $N(C_1-C_6)$, $CO_2(C_1-C_6)$ substituiert ist; einer $CH_2Br$-Gruppe;
wobei **W** ein Heteroatom, ausgewählt aus N, O und S, vorzugsweise O und noch bevorzugter S ist,

- der Verbindung der Formel (II):

[Chem. 2]

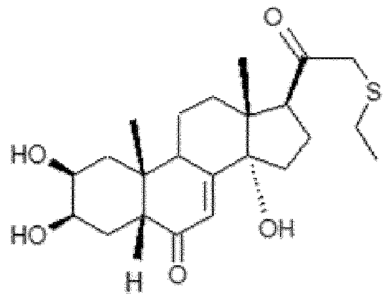

(II)

.

**2.** Zusammensetzung zu ihrer Verwendung nach Anspruch 1, die 20-Hydroxyecdyson umfasst.

**3.** Zusammensetzung zu ihrer Verwendung nach Anspruch 2, wobei das 20-Hydroxyecdyson in Form eines Pflanzenextrakts oder eines Pflanzenteils vorliegt, wobei die Pflanze aus Pflanzen ausgewählt ist, die auf das Trockengewicht der Pflanze bezogen mindestens 0,5 % 20-Hydroxyecdyson enthalten, wobei der Extrakt mindestens 95 %, und vorzugsweise mindestens 97 % 20-Hydroxyecdyson umfasst.

4. Zusammensetzung zu ihrer Verwendung nach Anspruch 3, die auf das Trockengewicht des Extrakts bezogen bemerkenswerterweise zwischen 0 und 0,05 % Verunreinigungen umfasst, die die Unbedenklichkeit, Verfügbarkeit oder Wirksamkeit einer pharmazeutischen Anwendung des Extrakts beeinflussen können.

5. Zusammensetzung zu ihrer Verwendung nach einem der Ansprüche 3 bis 4, wobei die Pflanze ausgewählt ist aus *Stemmacantha carthamoides, Cyanotis arachnoidea* und *Cyanotis vaga.*

6. Zusammensetzung zu ihrer Verwendung nach einem der Ansprüche 1 bis 5, wobei die Veränderung der Atemfunktion auf eine neuromuskuläre Erkrankung der Motoneuronen und/oder der neuromuskulären Verbindungsstelle und/oder der quergestreiften Skelettmuskulatur zurückgeht.

7. Zusammensetzung zu ihrer Verwendung nach einem der Ansprüche 1 und 6, wobei die Veränderung der Atemfunktion mit einer Veränderung der quergestreiften Muskulatur und/oder der glatten Muskulatur zusammenhängt.

8. Zusammensetzung zu ihrer Verwendung nach einem der Ansprüche 1 bis 5, wobei die Hyperreaktivität der Bronchien mit der Funktion der glatten Muskulatur der Bronchien assoziiert ist.

9. Zusammensetzung zu ihrer Verwendung nach einem der Ansprüche 1 bis 8, wobei die Veränderung der Atemfunktion mit einer Beeinträchtigung mindestens eines der Atemparameter zusammenhängt, ausgewählt aus dem Penh-Wert, der maximalen Einatmungsspitze, der maximalen Ausatmungsspitze, der Relaxationszeit und der Atemfrequenz.

10. Zusammensetzung zu ihrer Verwendung nach einem der Ansprüche 1 bis 9, wobei die Veränderung der Atemfunktion mit einer Beeinträchtigung mindestens eines der mechanischen Parameter des Lungengewebes zusammenhängt.

11. Zusammensetzung zu ihrer Verwendung nach Anspruch 10, wobei die Veränderung der Atemfunktion mit einer Abnahme der Lungencompliance und/oder einer Zunahme des Lungenwiderstands und/oder einer Abnahme der Lungenelastanz zusammenhängt.

12. Zusammensetzung zu ihrer Verwendung nach einem der Ansprüche 1 bis 11, wobei die Phytoecdysone beim Menschen in einer Dosis im Bereich zwischen 3 und 15 Milligramm pro Kilogramm pro Tag verabreicht werden.

13. Zusammensetzung zu ihrer Verwendung nach einem der Ansprüche 1 bis 12, wobei die Phytoecdysone bei einem Menschen im Erwachsenenalter in einer Dosis von 200 bis 1000 mg/Tag, in einer oder mehreren Gaben, und bei einem Menschen im Kindes- oder Säuglingsalter einer Dosis von 5 bis 350 mg/Tag, in einer oder mehreren Gaben, verabreicht werden.

14. Zusammensetzung zu ihrer Verwendung nach einem der Ansprüche 1 bis 13, wobei die mindestens eine Verbindung der allgemeinen Formel (I) ausgewählt ist aus:

- **Nr. 1:** (2S,3R,5R,10R,13R,14S,17S)-2,3,14-Trihydroxy-10,13-dimethyl-17-(2-morpholinoacetyl)-2,3,4,5,9,11,12,15,16,17-decahydro-1H-cyclopenta[a]phenanthren-6-on;
- **Nr. 2:** (2S,3R,5R,10R,13R,14S,17S)-2,3,14-Trihydroxy-17-[2-(3-hydroxypyrrolidin-1-yl)acetyl]-10,13-dimethyl-2,3,4,5,9,11,12,15,16,17-decahydro-1H-cyclopenta[a]phenanthren-6-on;
- **Nr. 3:** (2S,3R,5R,10R,13R,14S,17S)-2,3,14-Trihydroxy-17-[2-(4-Hydroxy-1-piperidyl)acetyl]-10,13-dimethyl-2,3,4,5,9,11,12,15,16,17-decahydro-1H-cyclopenta[a]phenanthren-6-on;
- **Nr. 4:** (2S,3R,5R,10R,13R,14S,17S)-2,3,14-Trihydroxy-17-[2-[4-(2-hydroxyethyl)-1-piperidyl]acetyl]-10,13-dimethyl-2,3,4,5,9,11,12,15,16,17-decahydro-1H-cyclopenta[a]phenanthren-6-on;
- **Nr. 5:** (2S,3R,5R,10R,13R,14S,17S)-17-[2-(3-Dimethylaminopropyl(methyl)amino)acetyl]-2,3,14-trihydroxy-10,13-dimethyl-2,3,4,5,9,11,12,15,16,17-decahydro-1H-cyclopenta[a]phenanthren-6-on;
- **Nr. 6:** 2-[2-Oxo-2-[(2S,3R,5R,10R,13R,14S,17S)-2,3,14-trihydroxy-10,13-dimethyl-6-oxo-2,3,4,5,9,11,12,15,16,17-decahydro-1H-cyclopenta[a]phenanthren-17-yl]ethyl]sulfanylethylacetat;
- **Nr. 7:** (2S,3R,5R,10R,13R,14S,17S)-17-(2-Ethylsulfanylacetyl)-2,3,14-trihydroxy-10,13-dimethyl-2,3,4,5,9,11,12,15,16,17-decahydro-1H-cyclopenta[a]phenanthren-6-on;
- **Nr. 8:** (2S,3R,5R,10R,13R,14S,17S)-2,3,14-Trihydroxy-17-[2-(2-hydroxyethylsulfanyl)acetyl]-10,13-dimethyl-2,3,4,5,9,11,12,15,16,17-decahydro-1H-cyclopenta[a]phenanthren-6-on.

## Claims

1. Composition comprising at least one phytoecdysone and/or at least one semi-synthetic derivative of phytoecdysone, for use thereof in mammals in the treatment of impaired respiratory function resulting from an acquired or genetic neuromuscular disease, or impaired respiratory function linked to bronchial hyperreactivity, said at least one semi-synthetic derivative of phytoecdysone being selected from among:

- a compound of general formula (I):

[Chem. 1]

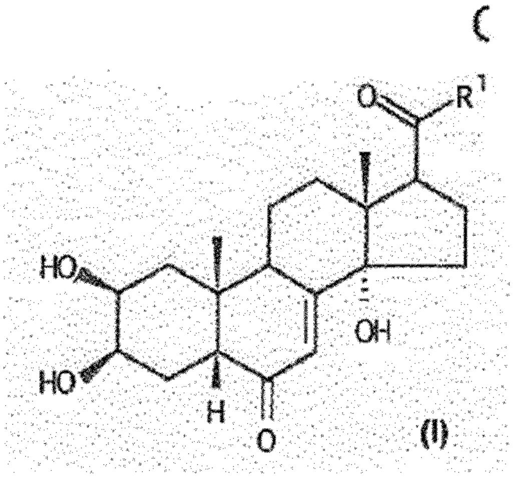

(I)

wherein:

> **$R^1$** is chosen from: a ($C_1$-$C_6$)**W**($C_1$-$C_6$) group; a ($C_1$-$C_6$)**W**($C_1$-$C_6$)**W**($C_1$-$C_6$) group; a ($C_1$-$C_6$)**W**($C_1$-$C_6$)$CO_2$($C_1$-$C_6$) group; a ($C_1$-$C_6$)**A** group, **A** representing a heterocycle optionally substituted by a group of type OH, MeO, ($C_1$-$C_6$), N($C_1$-$C_6$), $CO_2$($C_1$-$C_6$); a $CH_2Br$ group;
> **W** being a heteroatom chosen from N, O and S, preferably O and still more preferably S;

- the compound of formula (II):

[Chem. 2]

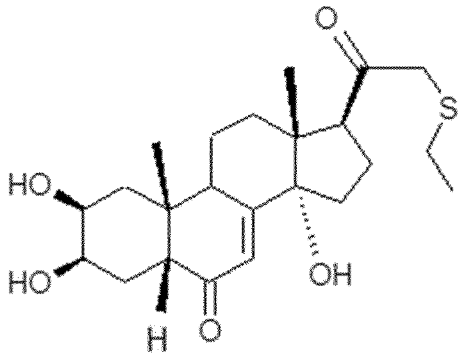

(II)

2. Composition for use thereof according to claim 1, including 20-hydroxyecdysone.

3. Composition for use thereof according to claim 2, wherein the 20-hydroxyecdysone is in the form of an extract from a plant or from a plant part, said plant being chosen from plants containing at least 0.5% 20-hydroxyecdysone by dry weight of said plant, said extract including at least 95%, and preferably at least 97%, of 20-hydroxyecdysone.

4. Composition for use thereof according to claim 3, notably including between 0 and 0.05%, by dry weight of the extract, of impurities which may affect the safety, availability or efficacy of a pharmaceutical application of said extract.

5. Composition for use thereof according to any one of claims 3 to 4, wherein the plant is chosen from *Stemmacantha carthamoides, Cyanotis arachnoidea* and *Cyanotis vaga.*

6. Composition for use thereof according to any one of claims 1 to 5, wherein the impaired respiratory function results from neuromuscular disease of the motoneurons and/or of the neuromuscular junction and/or of the striated skeletal muscle.

7. Composition for use thereof according to any one of claims 1 to 6, wherein the impaired respiratory function is linked to an impairment of the striated muscle and/or smooth muscle.

8. Composition for use thereof according to any one of claims 1 to 5, wherein the bronchial hyperreactivity is associated with bronchial smooth muscle function.

9. Composition for use thereof according to any one of claims 1 to 8, wherein the impaired respiratory function is linked to a condition of at least one of the respiratory parameters chosen from the Penh value, the maximum inspiratory peak, the maximum expiratory peak, relaxation time, and respiratory rate.

**10.** Composition for use thereof according to any one of claims 1 to 9, wherein the impaired respiratory function is linked to a condition of at least one of the mechanical parameters of the lung tissue.

**11.** Composition for the use thereof according to claim 10, wherein the impaired respiratory function is linked to a reduction in pulmonary compliance and/or an increase in pulmonary resistance and/or a reduction in pulmonary elastance.

**12.** Composition for use thereof according to any one of claims 1 to 11, wherein the phytoecdysones are administered in a dose between 3 and 15 milligrams per kilogram per day in humans.

**13.** Composition for use thereof according to any one of claims 1 to 12, wherein the phytoecdysones are administered in a dose of 200 to 1,000 mg/day, in one or more intakes, in an adult human, and a dose of 5 to 350 mg/day, in one or more intakes, in a human child or infant.

**14.** Composition for the use thereof according to any one of claims 1 to 13, wherein said at least one compound of general formula (I) is chosen from:

- **No. 1:** (2S,3R,5R,10R,13R,14S,17S)-2,3,14-trihydroxy-10,13-dimethyl-17-(2-morpholinoacetyl)-2,3,4,5,9,11,12,15,16,17-decahydro-1H-cyclopenta[a]phenanthren-6-one;
- **No. 2:** (2S,3R,5R,10R,13R,14S,17S)-2,3,14-trihydroxy-17-[2-(3-hydroxypyrrolidin-1-yl)acetyl]-10,13-dimethyl-2,3,4,5,9,11,12,15,16,17-decahydro-1H-cyclopenta[a]phenanthren-6-one;
- **No. 3:** (2S,3R,5R,10R,13R,14S,17S)-2,3,14-trihydroxy-17-[2-(4-hydroxy-1-piperidyl)acetyl]-10,13-dimethyl-2,3,4,5,9,11,12,15,16,17-decahydro-1H-cyclopenta[a]phenanthren-6-one;
- **No. 4:** (2S,3R,5R,10R,13R,14S,17S)-2,3,14-trihydroxy-17-[2-[4-(2-hydroxyethyl)-1-piperidyl]acetyl]-10,13-dimethyl-2,3,4,5,9,11,12,15,16,17-decahydro-1H-cyclopenta[a]phenanthren-6-one;
- **No. 5:** (2S,3R,5R,10R,13R,14S,17S)-17-[2-(3-dimethylaminopropyl(methyl)amino)acetyl]-2,3,14-trihydroxy-10,13-dimethyl-2,3,4,5,9,11,12,15,16,17-decahydro-1H-cyclopenta[a]phenanthren-6-one;
- **No. 6:** ethyl 2-[2-oxo-2-[(2S,3R,5R,10R,13R,14S,17S)-2,3,14-trihydroxy-10,13-dimethyl-6-oxo-2,3,4,5,9,11,12,15,16,17-decahydro-1H-cyclopenta[a]phenanthren-17-yl]ethyl]sulfanylacetate;
- **No. 7:** (2S,3R,5R,10R,13R,14S,17S)-17-(2-ethylsulfanylacetyl)-2,3,14-trihydroxy-10,13-dimethyl-2,3,4,5,9,11,12,15,16,17-decahydro-1H-cyclopenta[a]phenanthren-6-one;
- **No. 8:** (2S,3R,5R,10R,13R,14S,17S)-2,3,14-trihydroxy-17-[2-(2-hydroxyethylsulfanyl)acetyl]-10,13-dimethyl-2,3,4,5,9,11,12,15,16,17-decahydro-1H-cyclopenta[a]phenanthren-6-one.

[Fig. 1A]

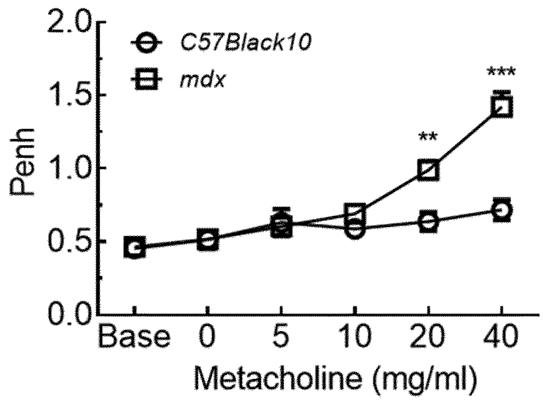


[Fig. 1B]

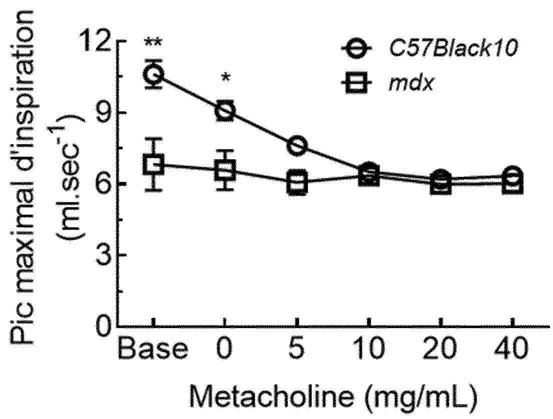


[Fig. 1C]

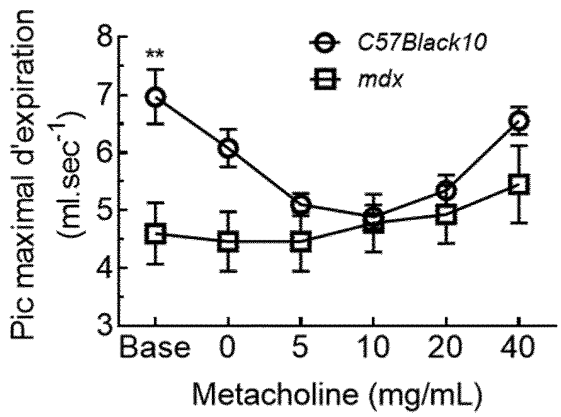

[Fig. 1D]

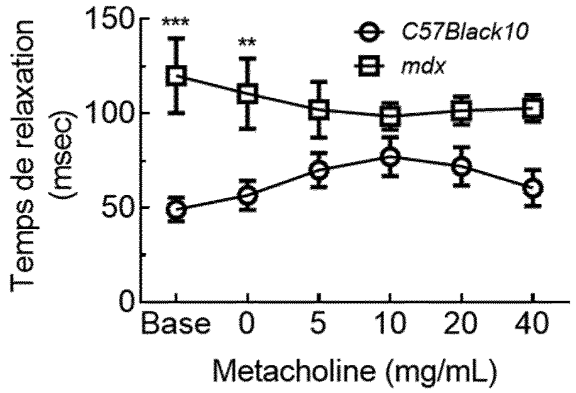


[Fig. 1E]

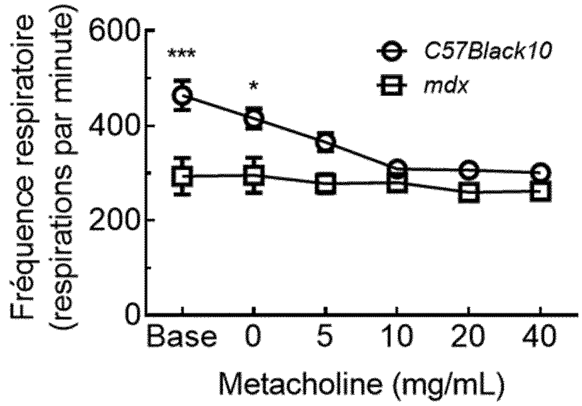


[Fig. 2A]

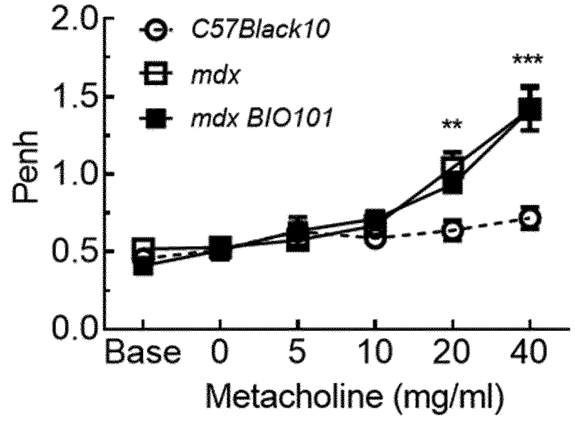

[Fig. 2B]

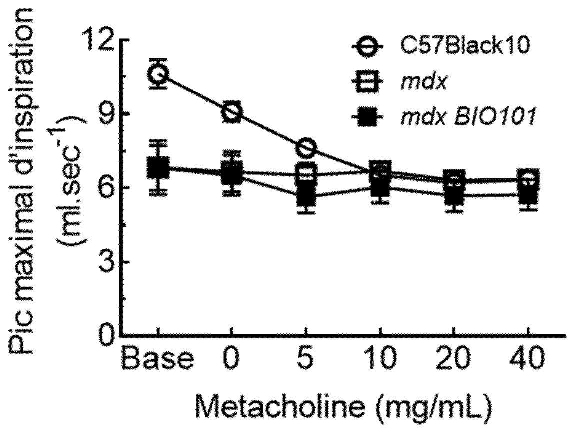


[Fig. 2C]

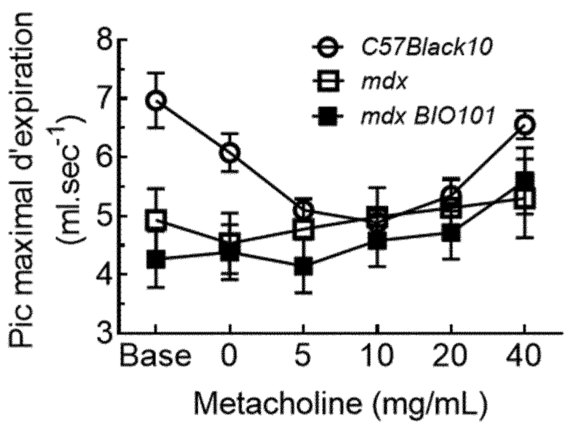


[Fig. 2D]

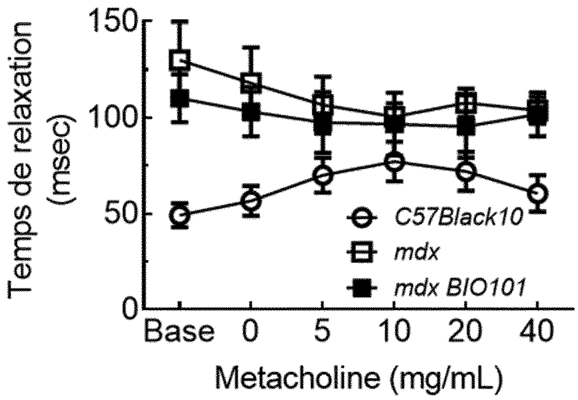

[Fig. 2E]

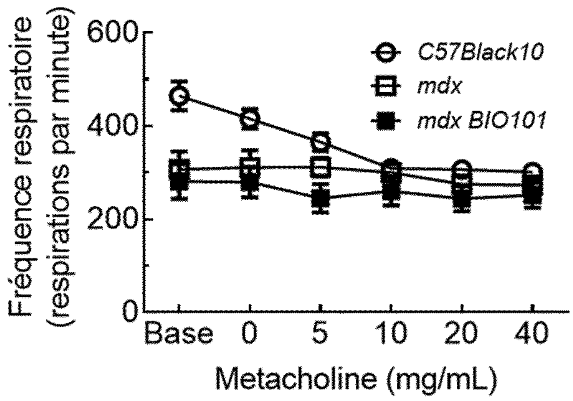


[Fig. 3A]

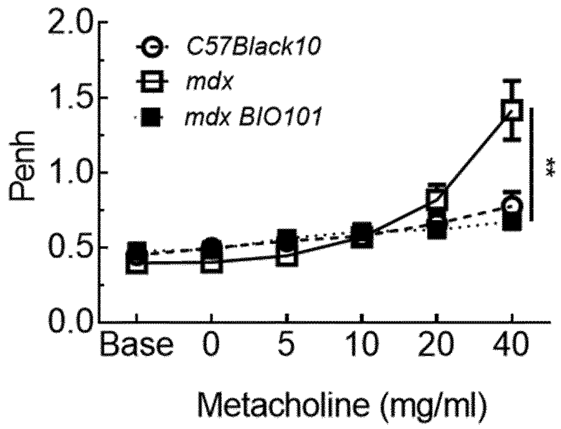


[Fig. 3B]

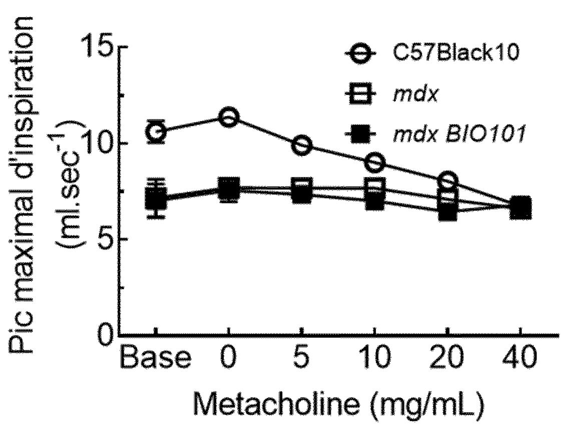

[Fig. 3C]

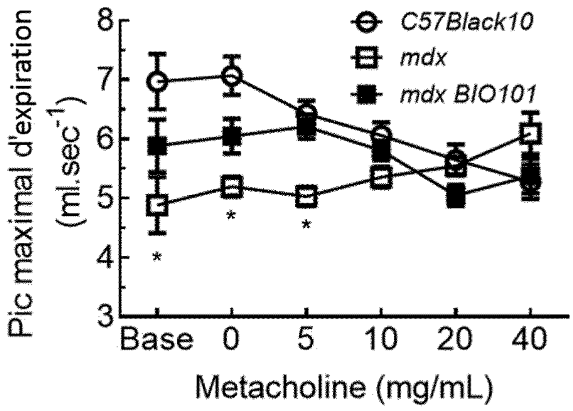


[Fig. 3D]

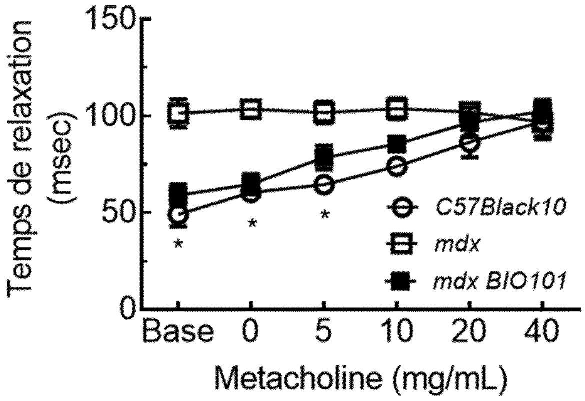


[Fig. 3E]

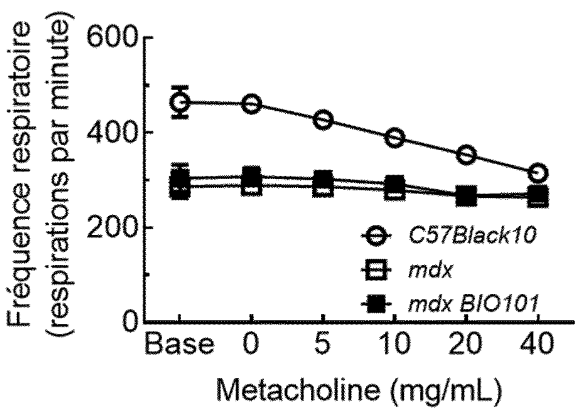

[Fig. 4A]

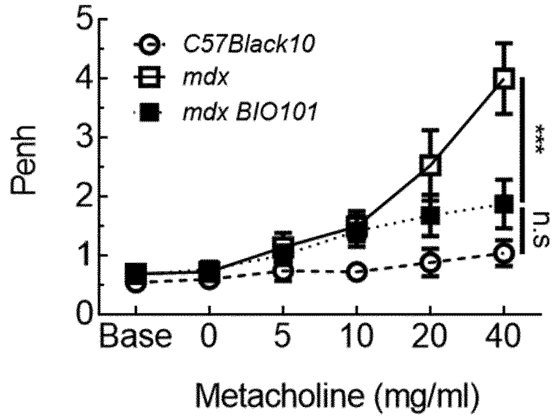


[Fig. 4B]

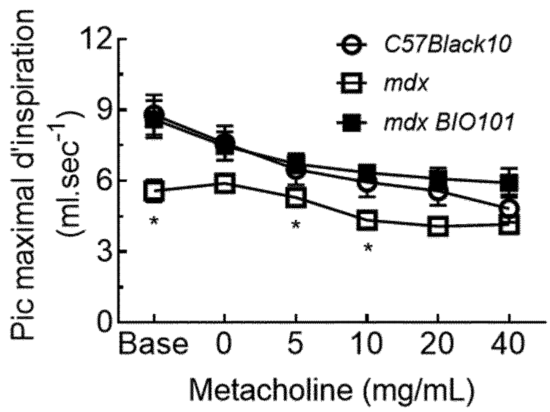


[Fig. 4C]

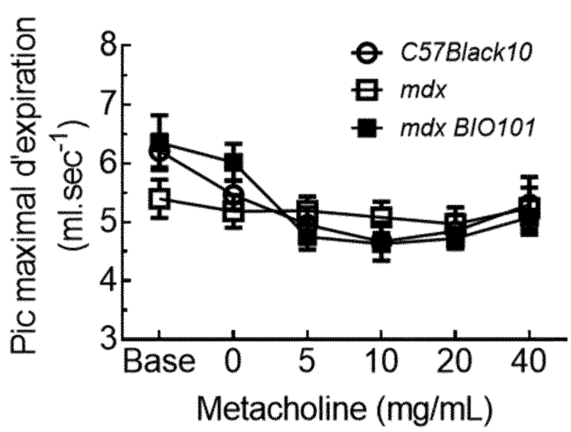

[Fig. 4D]

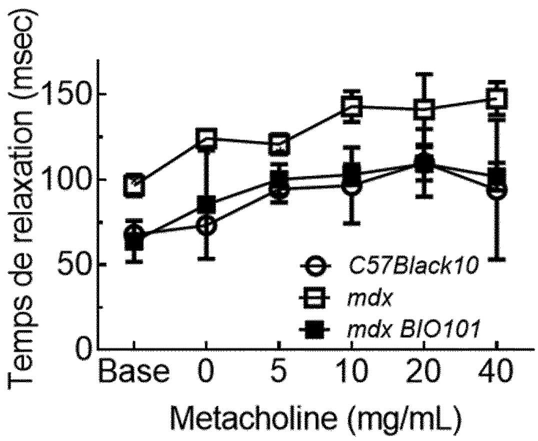


[Fig. 4E]

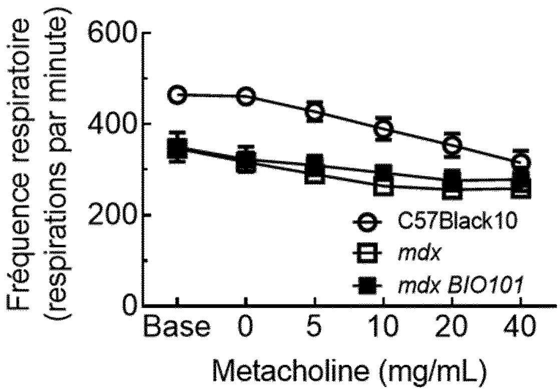


[Fig. 5A]

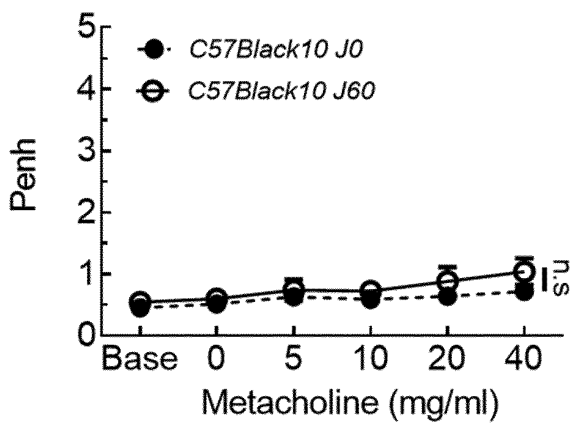

[Fig. 5B]

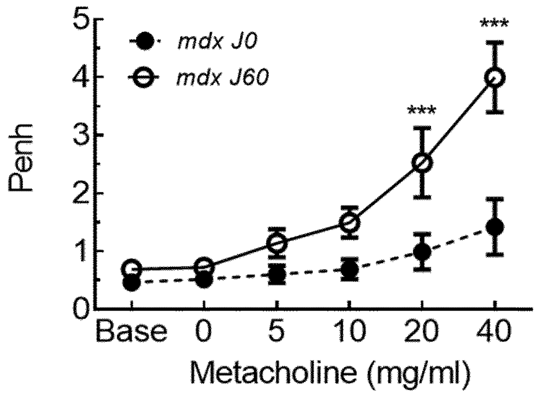


[Fig. 6A]

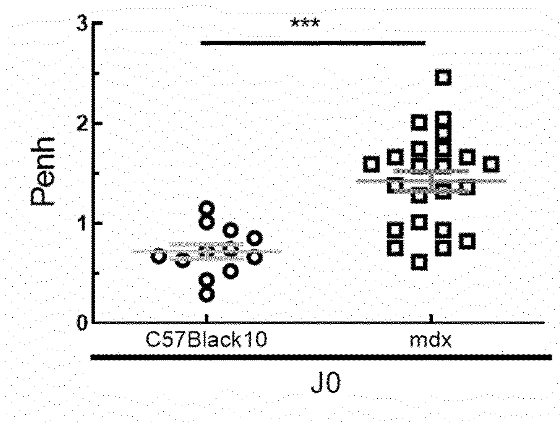


[Fig. 6B]

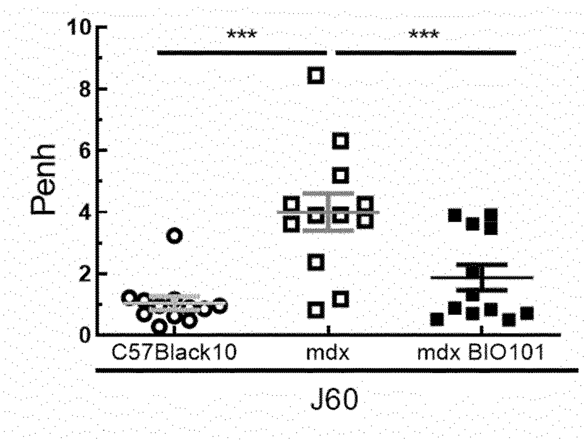

[Fig. 7A]

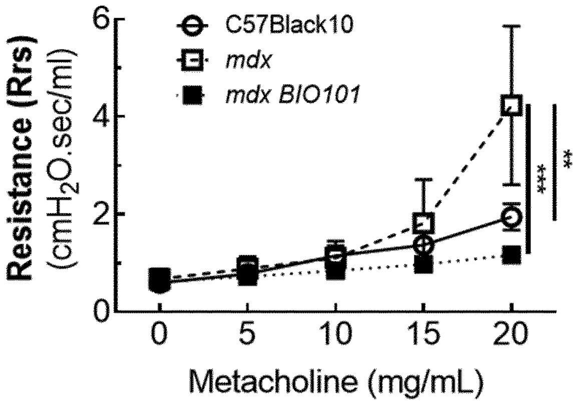


[Fig. 7B]

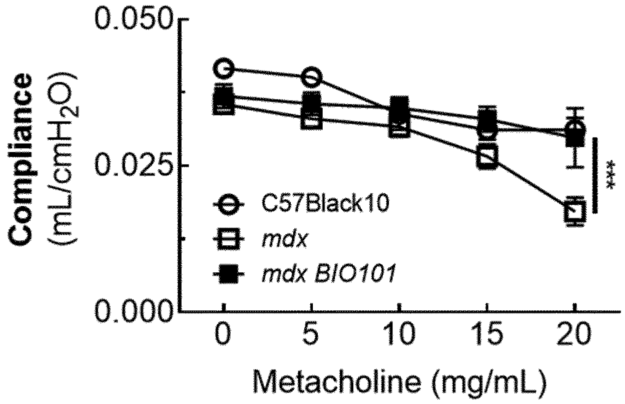


[Fig. 7C]

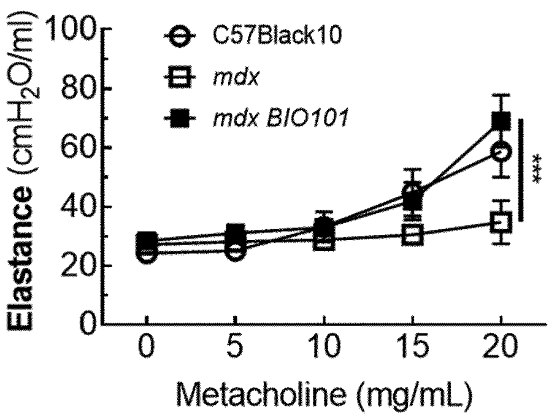

[Fig. 8A]

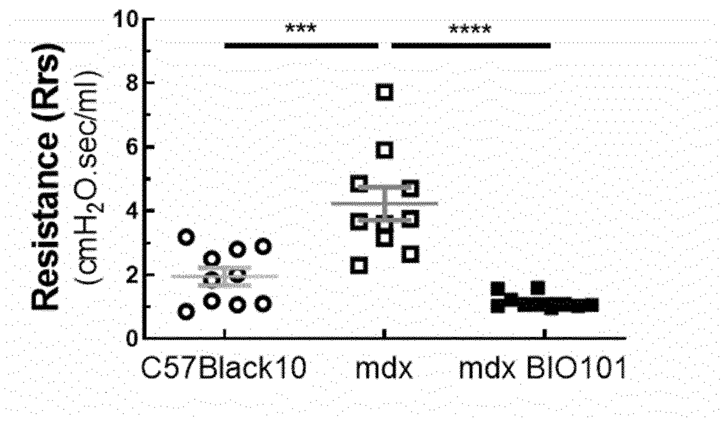


[Fig. 8B]

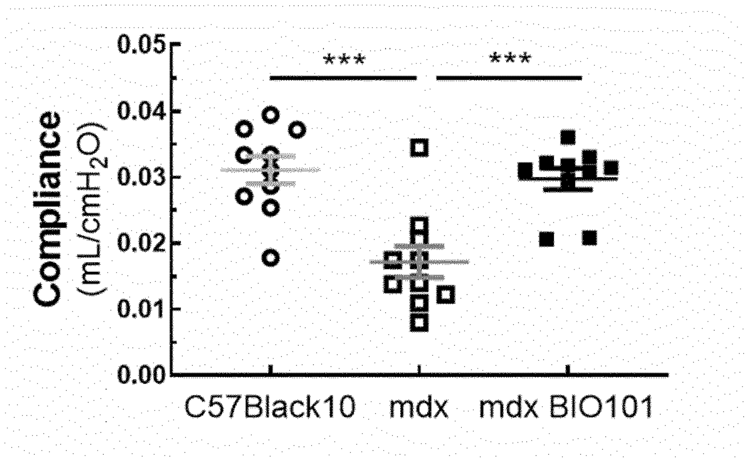


[Fig. 8C]

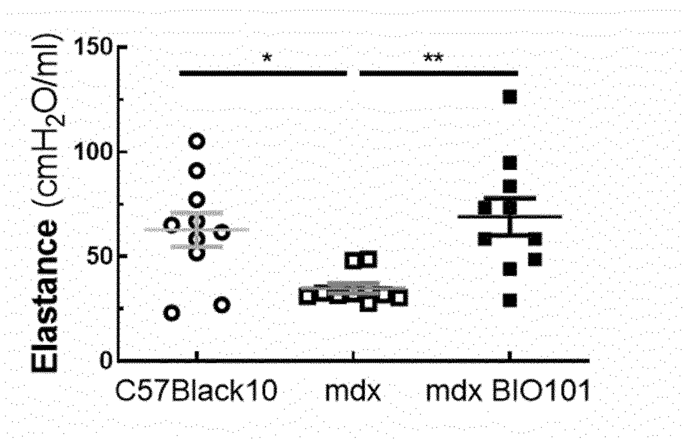

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- RU 2257906
- EP 15732785


### Littérature non-brevet citée dans la description


- **WU X et al.** *CHINESE PHARMACOLOGICAL BULLETIN,* 1998, vol. 14 (3), 256-258
- **WU XU et al.** *CHINESE JOURNAL OF TRAUMATOLOGY,* Mai 1997, 295-296
- **ADLER A ; CIESLEWICZ G ; IRVIN CG.** Unrestrained plethysmography is an unreliable measure of airway responsiveness in BALB/c and C57BL/6 mice. *J. Appl. Physiol. (1985,* 2004, vol. 97 (1), 286-92
- **BAYDUR A ; GILGOFF I ; PRENTICE W ; CARLSON M ; FISCHER DA.** Decline in respiratory function and expérience with long-term assisted ventilation in advanced Duchenne's muscular dystrophy. *Chest,* 1990, vol. 97, 884-9
- **BENDITT JO ; BOITANO LJ.** Pulmonary Issues in Patients with Chronic Neuromuscular Disease. *Am. J. Respir. Crit. Care Med.,* 2013, vol. 187 (10), 1046-55
- **BERGREN DR.** Chronic tobacco smoke exposure increases airway sensitivity to capsaicin in awake guinea pigs. *j. Appl. Physiol.,* 2001, vol. 90, 695-704
- **BIRNKRANT DJ ; PANITCH HB ; BENDITT JO ; BOITANO LJ ; CARTER ER et al.** American College of Chest Physicians consensus statement on the respiratory and related management of patients with Duchenne muscular dystrophy undergoing anesthesia or sedation. *Chest,* 2007, vol. 132, 1977-86
- **CHONG BT ; AGRAWAL DK ; ROMERO FA ; TOWNLEY RG.** Measurement of bronchoconstriction using whole-body plethysmograph: comparison of freely moving versus restrained guinea pigs. *J. Pharmacol. Toxicol. Methods,* 1998, vol. 39, 163-8
- **DJURIC VJ ; COX G ; OVERSTREET DH ; SMITH I ; DRAGOMIR A ; STEINER M.** Genetically transmitted cholinergic hyperresponsiveness predisposes to experimental asthma. *Brain Behav. Immun.,* 1998, vol. 12, 272-84
- **FINDER JD ; BIRNKRANT D ; CARL J ; FARBER HJ ; GOZAL D et al.** American Thoracic Society. Respiratory care of the patient with Duchenne muscular dystrophy: ATS consensus statement. *Am. J. Respir. Crit. Care Med.,* 2004, vol. 170, 456-65
- **GAYRAUD J ; MATECKI S ; HNIA K ; MORNET D ; PREFAUT C et al.** Ventilation during airbreathing and in response to hypercapnia in 5 and 16 month-old mdx and C57 mice. *J. Muscle Res. Cell Motil.,* 2007, vol. 28 (1), 29-37
- **GOSSELIN LE ; BARKLEY JE ; SPENCER MJ ; MCCORMICK KM ; FARKAS GA.** Ventilatory dysfunction in mdx mice: impact of tumor necrosis factor-alpha deletion. *Muscle Nerve,* 2003, vol. 28, 336-43
- **HAMELMANN E ; SCHWARZE J ; TAKEDA K ; OSHIBA A ; LARSEN GL ; IRVIN CG ; GELFAND EW.** Noninvasive measurement of airway responsiveness in allergic mice using barometric plethysmography. *Am. J. Respir. Crit. Care Med.,* 1997, vol. 156, 766-75
- **HOFFMAN AM ; DHUPA N ; CIMETTI L.** Airway reactivity measured by barometric whole-body plethysmography in healthy cats. *Am. J. Vet. Res.,* 1999, vol. 60, 1487-92
- **HUANG P ; CHENG G ; LU H ; ARONICA M ; RANSOHOFF RM ; ZHOU L.** Impaired respiratory function in mdx and mdx/utrn(+/-) mice. *Muscle Nerve,* 2011, vol. 43 (2), 263-7
- **INKLEY SR ; OLDENBURG FC ; VIGNOS PJ JR.** Pulmonary function in Duchenne muscular dystrophy related to stage of disease. *Am. J. Med.,* 1974, vol. 56, 297-306
- **ISHIZAKI M ; SUGA T ; KIMURA E ; SHIOTA T ; KAWANO R et al.** Mdx respiratory impairment following fibrosis of the diaphragm. *Neuromuscul. Disord.,* 2008, vol. 18 (4), 342-8
- **LO MAURO A ; ALIVERTI A.** Physiology of respiratory disturbances in muscular dystrophies. *Breathe,* 2016, vol. 12 (4), 318-27
- **MATECKI S ; RIVIER F ; HUGON G ; KOECHLIN C et al.** The effect of respiratory muscle training with CO2 breathing on cellular adaptation of mdx mouse diaphragm. *Neuromuscul. Disord.,* 2005, vol. 15, 427-36
- **MAYER OH ; FINKEL RS ; RUMMEY C ; BENTON MJ ; GLANZMAN AM et al.** Characterization of Pulmonary Function in Duchenne Muscular Dystrophy. *Pediatr. Pulmonol.,* 2015, vol. 50, 487-94

- **MCKIM DA ; ROAD J ; AVENDANO M ; ABDOOL S ; COTE F et al.** Canadian Thoracic Society Home Mechanical Ventilation Committee. Home mechanical ventilation: a Canadian Thoracic Society clinical practice guideline. *Can. Respir. J.,* 2011, vol. 18, 197-215
- **MILLER RG ; JACKSON CE ; KASARSKIS EJ ; ENGLAND JD ; FORSHEW D et al.** Quality Standards Subcommittee of the American Academy of Neurology. *Practice parameter update: the care of the patient with amyotrophic lateral sclerosis: drug, nutritional, and respiratory thérapies (an evidence-based review): report of the Quality Standards Subcommittee of the American Academy of Neurology. Neurology,* 2009, vol. 73, 1218-26
- **MOSQUEIRA M ; BABY SM ; LAHIRI S ; KHURANA TS.** Ventilatory chemosensory drive is blunted in the mdx mouse model of Duchenne Muscular Dystrophy (DMD). *PLoS One.,* 2013, vol. 8 (7), e69567
- **ONCLINX C.** Relation entre la résistance pulmonaire totale et la Penh en fonction de la localisation anatomique de l’obstruction des voies aériennes (mémoire-diplôme d’étude approfondie). Université de Liège, Faculté de médecine vétérinaire, 2003, 24
- **SMITH PE ; CALVERLEY PM ; EDWARDS RH ; EVANS GA ; CAMPBELL EJ.** Practical problems in the respiratory care of patients with muscular dystrophy. *N. Engl. J. Med.,* 1987, vol. 316, 1197-205
- **VIANELLO A ; BEVILACQUA M ; SALVADOR V ; CARDAIOLI C ; VINCENTI E.** Long-term nasal intermittent positive pressure ventilation in advanced Duchenne’s muscular dystrophy. *Chest,* 1994, vol. 105, 445-8